# EUROPÄISCHE PATENTSCHRIFT

(11) **EP 3 117 787 B1**
(45) Veröffentlichungstag und Bekanntmachung des Hinweises auf die Patenterteilung: **20.04.2022**
(21) Anmeldenummer: 16001248.0
(22) Anmeldetag: 02.06.2016
(51) Int. Cl.: A61B 17/70, A61B 17/86

(54) **PEDIKELSCHRAUBE MIT TULPE**
PEDICLE SCREW WITH TULIP
VIS PEDICULAIRE AVEC TULIPE

(30) Priorität: 09.06.2015 DE 102015007467; 23.06.2015 DE 102015008036
(43) Veröffentlichungstag der Anmeldung: 18.01.2017
(73) Patentinhaber: Signus Medizintechnik GmbH, 63755 Alzenau (DE)
(72) Erfinder: Bas, van der Pol, 63755 Alzenau (DE)
(74) Vertreter: Leinweber & Zimmermann

(56) Entgegenhaltungen:
- EP-A1- 2 335 625
- US-A1- 2010 198 272
- US-A1- 2013 204 263
- US-A1- 2014 163 619
- US-A1- 2014 214 084

## Beschreibung

Die Erfindung betrifft einen Instrumentensatz zum Verbinden von Wirbelkörpern mit einer in einen Pedikel einschraubbaren, einen Schraubenkopf und einen Schaft aufweisenden Schraube, einem mit der Schraube polyaxial verbindbaren Aufsatz zur Kopplung an ein Stabsystem mit einer hülsenförmigen Tulpe mit einer ersten Einführöffnung zum Einführen des Schraubenkopfes in einer Einführrichtung und einem Werkzeug, wobei der Aufsatz im Innenbereich der Tulpe einen Sattel aufweist, der Sattel drehfest in der Tulpe anordenbar ist und an einem der ersten Einführöffnung zugewandten ersten Endbereich einen Aufnahmebereich zum Aufnehmen des Schraubenkopfes und an einem dem Aufnahmebereich entgegengesetzten zweiten Endbereich einen mit dem Werkzeug koppelbaren Kopplungsbereich aufweist und zwischen einer durch Einführen des Schraubenkopfes erreichbaren Freigabelage und einer durch Verlagern des Schraubenkopfes aus der Freigabelage entgegen der Einführrichtung erreichbaren Sperrlage beweglich ist, wobei der in dem Aufnahmebereich aufgenommene Schraubenkopf in der Sperrlage nicht aus dem Aufnahmebereich lösbar ist, und der Sattel durch das an den Kopplungsbereich gekoppelte Werkzeug in einer das Freigeben des Schraubenkopfes aus dem Aufnahmebereich ermöglichenden Freigabelage fixierbar ist, so dass die Schraube und der Aufsatz voneinander trennbar sind.

Ein solcher Instrumentensatz ist aus dem Stand der Technik, beispielsweise aus der US 2010/198272 A1 bekannt und kommt beispielsweise bei Operationen der Wirbelsäule zum Einsatz, die eine Stabilisierung oder Korrektur der Wirbelsäule zum Ziel haben. Dabei werden zuerst Schrauben mit dem Schaft in die Pedikel der zu verbindenden benachbarten Wirbelkörper eingeschraubt. Jede Schraube ist mit ihrem Kopf mit je einem Aufsatz verbindbar. Danach wird ein üblicherweise starrer Verbindungsstab mit den Aufsätzen der Schrauben derart in longitudinaler Richtung gekoppelt, daß die Wirbel durch den Stab in der gewünschten Lage gehalten werden. Weiter ist ein Instrumentensatz bestehend aus einer Schraube und einer Tulpe mit einem Sattel aus der EP 2 335 625 A1 bekannt. Dahingegen offenbart die US 2014/0214084 A1 eine Alternative eines Instrumentensatzes, bei der nur eine Schraube und eine Tulpe, aber kein beweglicher Sattel verwendet werden. Auch die US 2014/0163619 A1 offenbart einen Instrumentensatz bestehend aus einer Schraube, einem Aufsatz mit einer Tulpe und einem Sattel sowie einem Werkzeug. Jedoch ist in diesem Fall die Schraube in einer Sperrlage des Sattels nur in einer Sagittalebene relativ zum Aufsatz drehbar, während in der Freigabelage die Drehung der Schraube auch in anderen Ebenen möglich ist.

Bei einem solchen operativen Eingriff ist die Kopplung des starren Verbindungsstabes an die Aufsätze eine Herausforderung. Aus dem Stand der Technik ist bekannt, daß sogenannte Polyaxialschrauben diesen Operationsschritt erheblich vereinfachen. Bei einer Polyaxialschraube bestehend aus einer Schraube und einem mit der Schraube verbundenen Aufsatz ist der Schaft der Schraube gegenüber der Längsachse des Aufsatzes in einem gewissen Bereich verschwenkbar. Weiter ist der Aufsatz in jeder Schwenklage des Schafts um seine Längsachse verdrehbar. Das Einsetzen des Verbindungsstabes in solche gegenüber dem Schaft der Schraube beweglichen Aufsätze ist wesentlich einfacher als im Fall von Aufsätzen, die starr mit dem Schaft der Schraube verbunden sind. Weiter erfordert ein starr mit dem Schaft verbundener Aufsatz eine Anordnung der Schraube in einem genau definierten, vorher bestimmten Winkel in dem Pedikel, um die gewünschte Ausrichtung der Wirbelkörper zu erhalten. Dahingegen erlauben die gegenüber dem Schaft beweglichen Aufsätze eine größere Toleranz beim Einsetzen der Schraube sowie eine Nachkorrektur der Lage der Wirbelköper vor der Arretierung des Stabsystems.

Üblicherweise wird bei einem aus dem Stand der Technik bekannten Stabsystem die Schraube mit dem an ihr befestigten Aufsatz zusammen in den Pedikel eingeschraubt. Dabei erweist sich jedoch die polyaxiale Verbindung des Aufsatzes und der Schraube als hinderlich. Zur Lösung dieses Problems wurde in der EP 2 208 473 A1 ein polyaxialer Schraubendreher offenbart. In der DE 2012 219 630 A1 ist in dem Schraubenkopf ein drehbares Antriebsprofil angeordnet, das derart auslenkbar ist, daß es zu jedem Zeitpunkt mit der Längsachse des Aufsatzes fluchtet.

Im Falle osteoporöser Wirbelkörper ist eine sichere, ein Herausbrechen verhindernde Fixierung der Schraube in den Knochen eine Herausforderung. Zudem soll im gesamten Operationsverlauf möglichst wenig Druck auf die Wirbelkörper ausgeübt werden. In der DE 102 46 177 A1 wird dazu in eine kanülierte Schraube nach dem Einsetzen ein Füllmaterial eingespritzt das durch in dem Schaft der Schraube angeordnete Fenster aus dem Schaft austritt und sich mit dem Knochen verbindet. Im Anschluß daran wird der Aufsatz über einen reibschlüssig mit dem Schaft verbindbaren Kopf an der Schraube fixiert. Ein Werkzeug zum Einspritzen von Füllmaterial in den Wirbelkörper wird beispielsweise in der US 2013/0204263 A1 offenbart.

Um einen schnellen und unkomplizierten Operationsverlauf zu ermöglichen, ist es wünschenswert, daß ein Instrumentensatz der eingangs genannten Art aus möglichst wenigen Einzelteilen besteht, welche einfach miteinander verbindbar sind.

Wie in der Eur.Spine J. 2010, Jan; 19 (1); 144 - 146 dargestellt wird, muß bei einer nicht geringen Anzahl an Patienten (5 bis 8 %) das Stabilisierungssystem nach dem operativen Eingriff jedoch wieder entfernt werden, da das Implantat entweder fehlerhaft ist oder aber der Patient negativ auf das Implantat reagiert.

Angesichts dieser Probleme im Stand der Technik liegt der Erfindung die Aufgabe zugrunde, einen Instrumentensatz der eingangs genannten Art derart weiterzubilden, daß die Belastung des Patienten beim Einsetzen und Entfernen der Schraube und des Aufsatzes in einen und aus einem Wirbelkörper verringert wird.

Erfindungsgemäß wird dieses Problem durch das Kennzeichen des Anspruchs 1 gelöst.

Ein erfindungsgemäßer Instrumentensatz besteht aus der Schraube, dem Aufsatz und dem Werkzeug und somit aus einer geringen Anzahl von Einzelteilen. Dies begünstigt einen schnellen Operationsverlauf. Die Schraube weist einen Schaft auf, der zweckmäßigerweise ein Gewinde zum Einschrauben in einen Pedikel in einer Einschraubrichtung aufweist. An einem der Einschraubrichtung entgegengesetzten Ende weist die Schraube einen Schraubenkopf auf, der mit dem Aufsatz verbindbar ist. Die Schraube wird ohne den Aufsatz in den Knochen eingeschraubt und der Aufsatz dann an der in dem Knochen fixierten Schraube befestigt. Das Einschrauben der Schraube wird somit durch den Aufsatz nicht behindert.

Der Aufsatz weist eine hülsenförmige Tulpe auf, in deren Innenbereich ein Sattel angeordnet ist. Der Schraubenkopf kann in eine Einführrichtung durch eine erste Einführöffnung der Tulpe in den Innenraum der Tulpe eingeführt werden.

Der Sattel weist an einem der Einführrichtung zugewandten ersten Endbereich einen Aufnahmebereich zum Aufnehmen des Schraubenkopfes auf. Der Aufsatz ist über den Sattel mit der Schraube koppelbar. Erfindungsgemäß ist die Verbindung des Schraubenkopfes und des Sattels derart ausgestaltet, daß der Schaft der Schraube gegenüber der Längsachse des Aufsatzes verschwenkbar ist und der Aufsatz in jeder Schwenklage des Schafts um seine Längsachse drehbar ist. Die Einheit aus Schraube und Aufsatz bildet also eine Polyaxialschraube.

An einem dem Aufnahmebereich entgegengesetzten zweiten Endbereich weist der Sattel einen Kopplungsbereich auf, der eine Kopplung mit dem Werkzeug ermöglicht. Das Werkzeug kann durch eine der ersten Einführöffnung entgegengesetzte zweite Einführöffnung der Tulpe an den Kopplungsbereich herangeführt werden und an diesen gekoppelt werden.

Der Sattel ist in der Tulpe in axialer Richtung zwischen einer Sperrlage und einer Freigabelage beweglich. Die Sperrlage ist dadurch definiert, daß der Schraubenkopf unlösbar mit dem Aufnahmebereich verbunden ist und dadurch der Aufsatz fest mit der Schraube gekoppelt ist. In der Freigabelage ist der Schraubenkopf mit dem Aufnahmebereich verbindbar und aus ihm lösbar.

Um die Schraube mit dem Aufnahmebereich zu verbinden muss der Sattel in der Freigabelage angeordnet werden. Dies kann beispielsweise dadurch erfolgen, daß der Schraubenkopf in der Einführrichtung durch die erste Einführöffnung in den Aufsatz eingeführt wird. Der Schraubenkopf kann dann gegen den Rand der Aufnahmeöffnung stoßen, und der Sattel kann von seiner Ausgangslage durch die Bewegung der Schraube in der Einführrichtung in die Freigabelage geschoben werden. Der Schraubenkopf kann aber auch, wie weiter unten erläutert wird, mittels des Werkzeugs in die Freigabelage bewegt werden. Der Sattel kann dann durch das an den Kopplungsbereich gekoppelte Werkzeug in der Freigabelage fixiert werden. Der Schraubenkopf ist dann in den Aufnahmebereich einsetzbar und mit dem Aufnahmebereich koppelbar.

Werden nun der Aufsatz und die Schraube voneinander weg bewegt, so kann der an den Schraubenkopf gekoppelte Sattel in die Sperrlage bewegt werden.

Zum Lösen des Schraubenkopfes aus dem Aufnahmebereich muß der Sattel in die Freigabelage bewegt werden. Dies kann, wie weiter unten erläutert wird, mit Hilfe des Werkzeugs erfolgen. Dann kann der Sattel durch das an dem Kopplungsbereich gekoppelte Werkzeug in der Freigabelage fixiert werden. Werden nun die Schraube und der Aufsatz voneinander weg bewegt, so fixiert das Werkzeug den Sattel weiter in der Freigabelage, und der Schraubenkopf kann aus dem Aufnahmebereich gelöst werden.

Die erfindungsgemäße Verbindung der Schraube und des Aufsatzes hält großen Kräften stand, was für eine der Stabilisierung der Wirbelsäule dienende Polyaxialschraube unabdingbar ist.

Die Kopplung und die Trennung des Aufsatzes und der in dem Pedikel fixierten Schraube kann mit Hilfe des Werkzeuges derart durchgeführt werden, daß nur eine geringe Kraft auf den Wirbelkörper ausgeübt wird. Dies ist insbesondere bei osteoporösen Knochen vorteilhaft.

Ein erfindungsgemäßer Instrumentensatz erlaubt ein schnelles und einfaches Lösen des Aufsatzes von der Schraube. Anders als bei vielen herkömmlichen Systemen kann die Schraube dabei im Knochen verbleiben. Dies vereinfacht den Austausch oder das Entfernen des Aufsatzes erheblich und kann auch zu einer Verkürzung der Operationszeit führen.

Eine weitere vorteilhafte Ausführungsform der Erfindung ist dadurch gekennzeichnet, daß der durch eine Wand begrenzte und eine Aufnahmeöffnung zum Einführen des Schraubenkopfes aufweisende Aufnahmebereich in einer quer zu einer Längsachse der Tulpe verlaufenden Querrichtung reversibel verformbar ist. Die Wand des Aufnahmebereichs und die Aufnahmeöffnung können dann in Richtung der Wand der Tulpe aufgeweitet werden und wieder in ihre ursprüngliche Form zurückgebildet werden. Die reversible Verformbarkeit des Aufnahmebereichs erlaubt eine Veränderung seiner Größe, wodurch die Aufnahme/das Lösen des Schraubenkopfes in den/aus dem Aufnahmebereich ermöglicht werden kann. Es kann/können die Wand und/oder die Aufnahmeöffnung reversibel verformbar sein.

Der Aufnahmebereich kann elastisch verformbar sein. Der Schraubenkopf kann dann in den Aufnahmebereich einrasten. Der Aufnahmebereich kann aber auch plastisch verformbar sein.

Die Wand des Aufnahmebereichs kann derart ausgestaltet sein, daß der Aufnahmebereich komplementär zu mindestens einem Bereich des Schraubenkopfes ausgebildet ist. Zumindest ein Bereich der Wand des Aufnahmebereichs und/oder der Aufnahmebereichöffnung kann/können zumindest einen Bereich des in dem Aufnahmebereich angeordneten Schraubenkopfes berühren. Eine Bewegung der Schraube kann so zu einer Bewegung des Sattels führen.

Vorteilhaft ist auch eine Ausführungsform, bei der die Wand des Aufnahmebereichs durch mindestens zwei Schlitze und/oder Fenster in mindestens zwei voneinander getrennte Bereiche unterteilt ist. Die Schlitze und/oder Fenster können eine Verformbarkeit auch bei einer Wahl eines harten Werkstoffs für die Wand des Sattels ermöglichen. Für den Sattel und die Tulpe kann dann derselbe Werkstoff verwendet werden. Dabei ist es vorteilhaft, wenn die Schlitze/Fenster den Rand der Aufnahmeöffnung mit einer Komponente senkrecht zur Aufnahmeöffnung durchbrechen. Dies begünstigt die Verformbarkeit bzw. Erweiterbarkeit der Aufnahmeöffnung in einer quer zur Einführrichtung verlaufenden Richtung. Die Wand kann durch die Schlitze/Fenster in zwei oder mehr Bereiche unterteilt werden. Mit einer steigenden Anzahl an getrennten Bereichen kann sich die Verformbarkeit des Aufnahmebereichs vergrößern. Jedoch kann sich dadurch die Festigkeit der Verbindung zwischen dem Schraubenkopf und dem Aufnahmebereich verringern. Vorteilhaft ist eine Ausführungsform, bei der die Wand in vier Bereiche unterteilt ist.

Bei einer vorteilhaften Ausführungsform der Erfindung können die Schlitze und/oder die Fenster T-förmig ausgebildet sein. Dabei ist es vorteilhaft, wenn sich der vertikale Balken des T von der Aufnahmeöffnung des Aufnahmebereichs im Wesentlichen senkrecht zur Aufnahmeöffnung erstreckt und dann in den horizontalen Balken des T übergeht, der sich dann im Wesentlichen parallel zur Aufnahmeöffnung bzw. in Umfangsrichtung des Sattels erstreckt. Dadurch weist die Wand in einem Abstand von der Aufnahmeöffnung einen Wandbereich auf, dessen Ausdehnung parallel zur Aufnahmeöffnung durch die horizontalen Balken zweier benachbarter T auf eine dünne Werkstoffbrücke verringert ist. Wird die Breite der Werkstoffbrücke verringert, so erhöht sich die Verformbarkeit des Aufnahmebereichs bzw. verringert sich die einer Verformung entgegenwirkende (Rückstell)kraft.

Bei einer weiteren möglichen Ausführung des Instrumentensatzes kann zumindest in der Sperrlage eine Wand der Tulpe eine Verformung des Aufnahmebereichs in der Querrichtung begrenzen. Dadurch kann der Schraubenkopf in der Sperrlage unlösbar mit dem Aufnahmebereich verbunden sein. Ist der Aufnahmebereich plastisch verformbar, so kann die Wand, welche die Verformung des Aufnahmebereichs in der Sperrlage begrenzt, den in der Freigabelage aufgeweiteten Aufnahmebereich in der Sperrlage in seine ursprüngliche Form zurückbilden. Die Wand der Tulpe kann auch in der Freigabelage eine Verformung des Aufnahmebereichs in der Querrichtung begrenzen. Sie kann aber auch in der Freigabelage eine ungehinderte Verformung des Aufnahmebereichs ermöglichen.

In einer weiteren vorteilhaften Ausführungsform der Erfindung kann der Schraubenkopf in einem ersten Kopfbereich in einer Richtung senkrecht zum Schaft eine maximale Breite BK aufweisen, und ein zwischen dem ersten Kopfbereich und dem Schaft angeordneter zweiter Kopfbereich kann in der Richtung senkrecht zum Schaft eine zweite Breite bK aufweisen, die kleiner als die maximale Breite BK ist. Der Schraubenkopf kann beispielsweise kugelförmig ausgebildet sein. Er kann aber auch polyederförmig ausgebildet sein. Bevorzugt weist der Schraubenkopf die Form einer Kugelschale auf. Bevorzugt ist der Aufnahmebereich dann komplementär zu einem Bereich der Kugelschale ausgebildet, welche sich zumindest von einem dem Schaft entgegengesetzten Endbereich des Schraubenkopfes bis zum zweiten Kopfbereich erstreckt. Dann ist der Schraubenkopf besonders gleichförmig in dem Aufnahmebereich drehbar. Die zweite Breite bK kann kleiner als die maximale Breite des Schafts sein. Sie kann aber auch größer als die maximale Breite des Schafts sein.

In einer zweckmäßigen Ausführungsform der Erfindung kann in der Sperrlage des Sattels die Wand der Tulpe eine durch eine Verformung in Querrichtung erreichbare maximale Ausdehnung der Aufnahmeöffnung auf eine Sperrausdehnung (DS) begrenzen, die kleiner als die maximale Breite (BK) und größer oder gleich der zweiten Breite (bK) des Schraubenkopfes ist, und in der Freigabelage des Sattels kann die Wand der Tulpe eine durch Verformung in Querrichtung erreichbare maximale Ausdehnung der Aufnahmeöffnung auf eine Freigabeausdehnung (DF) erlauben, die mindestens der maximalen Breite (BK) des Schraubenkopfes entspricht. Befindet sich der Sattel in der Sperrlage, und wird der Schraubenkopf durch die erste Einführöffnung in die Tulpe eingeführt, so stößt der Schraubenkopf in einem Bereich, der mit dem ersten Kopfbereich zusammenfallen kann, gegen die Aufnahmeöffnung des Aufnahmebereichs. Da die maximale Ausdehnung der Aufnahmeöffnung in der Sperrlage kleiner als die maximale Breite (BK) des ersten Kopfbereichs ist, wird der Sattel, wenn die Schraube weiter in der Einführrichtung bewegt wird, in Richtung der Freigabelage geschoben. Dort erlaubt die Wand der Tulpe eine Ausdehnung der Aufnahmeöffnung auf eine Ausdehnung (DF), die mindestens der maximalen Breite (BK) des Schraubenkopfes entspricht. Wird der Sattel nun in der Freigabelage beispielsweise durch das Werkzeug fixiert und die Schraube weiter in der Einführrichtung bewegt, so übt der Schraubenkopf Druck auf die Aufnahmeöffnung aus. Die Aufnahmeöffnung kann sich in der Freigabelage, ebenso wie die Wand des Aufnahmebereichs, in Richtung der Wand der Tulpe verformen, wodurch der Aufnahmebereich aufgeweitet wird. Die Aufnahmeöffnung kann sich auf eine Freigabeausdehnung (DF) ausdehnen, die mindestens der maximalen Breite (BK) des Schraubenkopfs entspricht. Dadurch kann der Schraubenkopf in den Aufnahmebereich eingeführt werden. Sind die Wand und die Aufnahmeöffnung des Aufnahmebereichs elastisch reversibel verformbar, so kann sich die Aufnahmeöffnung des Aufnahmebereichs nach Einführen des Schraubenkopfs wieder auf ihre ursprüngliche Größe verkleinern. Der Schraubenkopf ist dann zumindest teilweise von der Wand des Aufnahmebereichs umgeben in dem Aufnahmebereich des Sattels angeordnet. Ein Bereich des Schraubenkopfes kann den Rand der Aufnahmeöffnung berühren. Ist die zweite Breite (bK) gleich der Ausdehnung der Aufnahmeöffnung, so kann der zweite Bereich des Schraubenkopfes den Rand der Aufnahmeöffnung berühren. Wird nun die ggf. notwendige Fixierung des Sattels durch das Werkzeug in der Freigabelage gelöst und die Schraube entgegen der Einführrichtung bewegt, so kann ein Bereich des Schraubenkopfs mit der Aufnahmeöffnung in Berührung kommen, die Axialkraft kann auf den Sattel übertragen werden, und der Sattel wird von der Freigabelage in die Sperrlage bewegt. In der Sperrlage ist die maximale Ausdehnung der Aufnahmeöffnung auf die Sperrausdehnung (DS) begrenzt, die kleiner ist als die Breite (BK) des ersten Kopfbereichs. Folglich kann der Schraubenkopf nicht durch eine Bewegung der Schraube und des Aufsatzes in entgegengesetzter Richtung aus dem Aufnahmebereich gelöst werden.

In einer weiteren Ausführungsform der Erfindung kann ein Innendurchmesser (Dlmin) der Tulpe an der ersten Einführöffnung minimal sein und ein Innendurchmesser (Dlmax) der Tulpe in einer durch die Aufnahmeöffnung des Sattels in der Freigabelage definierten Ebene maximal sein. Die den Innenbereich der Tulpe begrenzende Wand kann dabei im Bereich der Freigabelage eine die Wand in geschlossener Kontur umlaufende Aussparung aufweisen. Die Wand kann aber auch im Bereich der Freigabelage mehrere voneinander getrennte Aussparungen aufweisen, welche eine Ausdehnung der Aufnahmeöffnung erlauben. Die den Innenbereich der Tulpe begrenzende Wand kann von der/den Aussparung/en konisch auf die erste Einführöffnung zulaufen.

Vorteilhaft ist auch eine Ausführungsform, bei der die Axialbewegung des Sattels in der Tulpe zwischen einer ersten Lage und einer zweiten Lage begrenzt ist. Dadurch wird verhindert, daß der Sattel aus der Tulpe herausfällt. Die erste und die zweite Lage können derart gewählt werden, daß sich der Sattel nur vollständig innerhalb der Tulpe bewegen kann.

Erfindungsgemäß kann die erste Lage mit der Freigabelage zusammenfallen. Dann kann der Schraubenkopf mit dem Aufnahmebereich verbunden werden, ohne daß der Sattel mit dem Werkzeug in der Freigabelage fixiert werden muß. Denn bei dieser Ausführungsform kann der in die erste Einführöffnung der Tulpe eingeführte Schraubenkopf den Sattel bis zur Freigabelage schieben. Da der Sattel in der Einführrichtung nur bis zur Freigabelage hin bewegbar ist, wird der Sattel durch die Bewegung der Schraube in der Einführrichtung in der Freigabelage fixiert, und der durch den Schraubenkopf auf die Aufnahmeöffnung ausgeübte Druck bewirkt eine Ausdehnung der Aufnahmeöffnung, so daß der Schraubenkopf in den Aufnahmebereich eingeführt werden kann.

Vorteilhaft ist auch eine Ausführungsform, bei der der Sattel zwischen dem Aufnahmebereich und dem Kopplungsbereich mindestens einen zur Wand hin hervorspringenden, in eine in der Wand der Tulpe ausgebildete Aussparung eingreifenden Vorsprung aufweist, wobei der bei einer Axialbewegung des Sattels an einen die Aussparung in axialer Richtung begrenzenden ersten und zweiten Anschlagsbereich anstoßende Vorsprung die Bewegung des Sattels zwischen der ersten und der zweiten Lage begrenzen kann. Die Wand kann dabei einen einzelnen Vorsprung aufweisen. Sie kann aber auch mehrere Vorsprünge aufweisen. Bevorzugt weist die Wand zwei sich diametral gegenüberliegende Vorsprünge auf. Der Vorsprung kann die Form eines Quaders aufweisen. Zu jedem Vorsprung ist in der Wand eine Aussparung angeordnet. Die Aussparung ist derart ausgebildet, daß jeder Vorsprung in die jeweilige Aussparung eingreifen kann. Jede Aussparung weist einen ersten und einen zweiten Anschlagsbereich auf, die jeweils eine Komponente senkrecht zur Einführöffnung aufweisen und die Ausdehnung der Aussparung in der Axialrichtung begrenzen. Wird der Sattel in der Einführrichtung bewegt, so stößt eine eine Komponente senkrecht zur Einführrichtung aufweisende erste Anschlagsfläche des Vorsprungs an den ersten Anschlagsbereich an, wodurch die Bewegung des Sattels in der Einführrichtung begrenzt wird. Dadurch wird die erste Lage definiert. Wird der Sattel entgegengesetzt zur Einführrichtung bewegt, so stößt eine eine Komponente senkrecht zur Einführrichtung aufweisende zweite Anschlagsfläche des Vorsprungs an den zweiten Anschlagsbereich an, wodurch die Bewegung des Sattels entgegengesetzt zur Einführrichtung begrenzt wird. Dadurch wird die zweite Lage definiert. Vorzugsweise sind die erste und die zweite Anschlagsfläche des Vorsprungs komplementär zu der Oberfläche des ersten bzw. zweiten Anschlagsbereichs ausgebildet. Dadurch ist die Kontaktfläche der an einen Anschlagsbereich anstoßenden Anschlagsfläche maximal.

Die Anordnung des Vorsprungs und der Aussparung kann aber auch vertauscht werden. So kann die Wand einen oder mehrere Vorsprünge aufweisen, welche in jeweils komplementäre Aussparungen des Sattels eingreifen können und so die Bewegung des Sattels in der Tulpe begrenzen.

In einer vorteilhaften Ausführungsform der Erfindung können die Tulpe und der Sattel miteinander lösbar verbunden sein. Die Tulpe und der Sattel können dann in verschiedenen Fertigungsanlagen hergestellt werden und entweder vom Hersteller oder vom Verbraucher miteinander verbunden werden.

In einer weiteren Ausführungsform der Erfindung kann die Tulpe an einem der ersten Einführöffnung entgegengesetzten zweiten Endbereich eine zweite Einführöffnung mit einem Randbereich aufweisen, in dem mindestens eine, vorzugsweise zwei sich diametral gegenüberliegende Einbuchtungen zur Aufnahme eines Stabes des Stabsystems ausgebildet sind. Der Stab kann dann von der zweiten Einführöffnung aus in die Einbuchtung(en) eingesetzt werden. Der Rand jeder Einbuchtung kann U-förmig ausgebildet sein. Dabei können sich die Schenkel des U von der Einführöffnung im Wesentlichen parallel zur Längsachse der Tulpe in Richtung der ersten Einführöffnung erstrecken und dann durch den bogenförmigen Teil des U miteinander verbunden werden.

Bei einem erfindungsgemäßen Instrumentensatz kann der Sattel an einem in Richtung der zweiten Einführöffnung der Tulpe angeordneten zweiten Endbereich eine sich diametral durch den Sattel erstreckende Aussparung zur Aufnahme des in den Einbuchtungen des Randbereichs der Tulpe angeordneten Stabes aufweisen. Die Aussparung kann U-förmig ausgebildet sein. Dabei können sich die Schenkel des U von dem zweiten Endbereich des Sattels im Wesentlichen parallel zur Einführrichtung auf den Aufnahmebereich zu erstrecken und dann vor Erreichen des Aufnahmebereichs in den bogenförmigen Teil des U übergehen. Die Krümmung des bogenförmigen Teils des U und der Abstand der Schenkel sind dabei derart ausgebildet, daß die Beweglichkeit eines in der Aussparung aufgenommenen zylinderförmigen Stabes in einer Richtung senkrecht zur Achse des Stabes und senkrecht zu einer Achse des Aufsatzes minimal ist.

Erfindungsgemäß kann die Tulpe an dem zweiten Endbereich eine Arretierungsvorrichtung zur Fixierung des Stabes aufweisen. Durch die Arretierungsvorrichtung kann der Stab derart an den Aufsätzen der in den Pedikeln eingeschraubten Schrauben befestigt werden, daß die entsprechenden Wirbel in der gewünschten Lage fixiert werden können. Die Arretierungsvorrichtung kann ein an dem zweiten Endbereich der Tulpe angeordnetes Innengewinde aufweisen. Das Innengewinde kann mit einer Madenschraube verschraubbar sein. Dies kann die gewünschte Arretierung des Stabes in dem Aufsatz ermöglichen.

Erfindungsgemäß weist das Werkzeug einen Haltegriff mit einem hohlen Innenraum und ein Kopplungsteil zur Kopplung an den Kopplungsbereich des Sattels auf, wobei das Kopplungsteil zumindest teilweise in dem Innenraum angeordnet und um eine Längsachse (A) drehbar ist. Das Kopplungsteil kann zylinderförmig ausgebildet sein. Weiter kann das Kopplungsteil an einem dem ersten Endbereich entgegengesetzten zweiten Endbereich einen Drehgriff aufweisen. Das Werkzeug kann an dem Haltegriff gehalten und geführt werden. Der Haltegriff kann Rillen und/oder Fenster aufweisen, wodurch die Griffigkeit des Haltegriffs erhöht werden kann. Das Kopplungsteil kann über den Drehgriff um seine Längsachse gedreht werden. Der Drehgriff kann Rillen und/oder Kanten aufweisen, die die Griffigkeit des Drehgriffs verbessern.

Erfindungsgemäß ist der Sattel drehfest in der Tulpe angeordnet. Der Kopplungsbereich des Sattels weist ein Innengewinde auf. Weiter weist das Kopplungsteil des Werkzeugs an einem ersten Endbereich ein Außengewinde zum Verschrauben mit dem Innengewinde des Kopplungsbereichs auf. Das Werkzeug weist weiter eine Arretierungsvorrichtung auf, mit der der Haltegriff drehfest mit der Tulpe verbindbar ist. Die Arretierungsvorrichtung kann zwei von dem Haltegriff hervorspringende, mit den Aussparungen der Tulpe in Eingriff bringbare Vorsprünge aufweisen. Die Vorsprünge können sich diametral gegenüberliegen. Weiter kann jeder Vorsprung eine U-förmige Kontur aufweisen. Dabei erstrecken sich die Schenkel des U zweckmäßigerweise von dem ersten Randbereich des Werkzeugs im Wesentlichen parallel zu der Längsachse des Werkzeugs und werden dann über den bogenförmigen Abschnitt des U miteinander verbunden. Diese Ausführungsform ist besonders günstig, wenn der an der zweiten Einführöffnung angeordnete Randbereich der Tulpe zwei sich gegenüberliegende U-förmige Einbuchtungen aufweist. Die U-förmigen Vorsprünge sind dann zweckmäßigerweise komplementär zu den U-förmigen Aussparungen ausgebildet.

Wird nun das Außengewinde des Kopplungsteils des Werkzeugs mit dem Innengewinde des Kopplungsbereichs des Sattels verschraubt, so kann, da der Sattel drehfest in der Tulpe angeordnet ist und weiter der Haltegriff des Werkzeugs drehfest mit der Tulpe gekoppelt ist, die Drehbewegung des Kopplungsteils in eine Axialbewegung des Sattels umgewandelt werden. Der Sattel kann somit von der Sperrlage in die Freigabelage bewegt werden. Der Sattel kann auch durch die Kopplung an das Kopplungsteil des Werkzeugs in der Freigabelage fixiert werden.

In einer weiteren Ausführungsform der Erfindung kann das Kopplungsteil gegenüber dem Haltegriff in axialer Richtung zwischen einer ersten Endlage und einer zweiten Endlage verschiebbar sein. In der ersten Endlage ist das Kopplungsteil in den Innenraum des Haltegriffs zurückgezogen. Das Kopplungsteil kann dabei so weit in den Innenraum des Werkzeugs zurückgezogen sein, daß der erste Endbereich mit dem Außengewinde vollständig in dem Innenraum angeordnet ist. In der zweiten Endlage kann das Kopplungsteil in axialer Richtung über einen in Richtung des Kopplungsteils angeordneten ersten Haltegriffendbereich des Haltegriffs hervorstehen. Das Kopplungsteil kann beispielsweise dadurch verschoben werden, daß das Werkzeug am Haltegriff gehalten wird und das Kopplungsteil am Drehgriff gehalten wird und dann in axialer Richtung bewegt wird. Dadurch kann das Einschrauben des Kopplungsteils in den Kopplungsbereich wie folgt vereinfacht werden: Das Werkzeug kann mit dem in der ersten Endlage angeordneten Kopplungsteil über die Arretierungsvorrichtung des Werkzeugs drehfest mit der Tulpe verbunden werden. Ist der erste Endbereich des Kopplungsteils vollständig in dem Innenraum des Werkzeugs angeordnet, kann das Werkzeug besonders einfach mit der Tulpe verbunden werden. Dann kann das Kopplungsteil in Richtung der zweiten Endlage und damit in Richtung der Tulpe geschoben werden, bis das Außengewinde des Kopplungsteils mit dem Innengewinde des Kopplungsbereichs des Sattels in Eingriff gebracht werden kann. Durch Drehen des Drehgriffs kann das Kopplungsteil dann in den Kopplungsbereich eingeschraubt werden. Wie bereits erläutert wurde, kann die Drehbewegung des Kopplungsteils in eine Axialbewegung des Sattels umgewandelt werden, und der Sattel kann in die Freigabelage bewegt werden.

Bei einem erfindungsgemäßen Instrumentensatz kann die Schraube massiv oder kanüliert und/oder gefenstert ausgebildet sein. Eine kanülierte Schraube kann mit Hilfe eines Führungsdrahts in den Knochen eingeschraubt werden. Dies kann die Positionierung der Schraube erleichtern. Weiter kann in eine kanülierte und gefensterte Schraube ein Füllmaterial in die Kanüle eingespritzt werden, das durch die Fenster austritt und sich mit dem Knochen verbindet. Dies kann eine Fixierung in einem osteoropösen Knochen erleichtern.

In einem Instrumentensatz gemäß der vorliegenden Erfindung werden bevorzugt Schrauben mit einer Schraubenlänge von 30 mm bis 100 mm verwendet. Bevorzugt werden Schrauben mit einem Schaftdurchmesser von 4,5 mm oder 5,5 mm oder 6,5 mm oder 7,5 mm oder 8,5 mm oder 9,5 mm verwendet.

Erfindungsgemäß kann die Schraube ein erstes Gewinde mit einer ersten Gewindesteigung und ein zweites Gewinde mit einer zweiten Gewindesteigung aufweisen, und die erste und die zweite Gewindesteigung können gleich oder verschieden sein. Die Verwendung verschiedener Gewindesteigungen kann es ermöglichen, Zug oder Druck auf einen Knochen auszuüben oder auch abgesplitterte Knochenstücke miteinander zu verbinden.

Die Schraube kann auch derart ausgebildet sein, daß die erste Gewindesteigung und die zweite Gewindesteigung gleich sind und die beiden Gewinde derart entlang des Schaftes der Schraube angeordnet sind, daß die Schraube in einem an den Schraubenkopf angrenzenden zweiten Schaftbereich zweigängig ist und in einem sich von dem zweiten Schaftbereich entgegengesetzt zum Schraubenkopf erstreckenden ersten Schaftbereich eingängig ist. Dadurch kann die Verankerung der Schraube im Knochen verbessert werden.

In einer weiteren Ausführungsform der Erfindung kann der Schraubenkopf eine Aussparung zur Aufnahme eines Schraubendrehers aufweisen. Die Aussparung kann als ein Innenvierkant, ein Innensechskant (Inbus), ein Innen-Sechsrund (Torx) oder ein Innen-Vielzahn (XZN) ausgebildet sein.

Im Rahmen dieser Beschreibung ist der Aufsatz gemeinsam mit der Schraube und dem Werkzeug beschrieben. Jedoch wird auch für den Aufsatz alleine, ohne die Schraube und das Werkzeug, Schutz begehrt.

Im Rahmen dieser Beschreibung sind der Aufsatz und die Schraube gemeinsam mit dem Werkzeug beschrieben. Jedoch wird auch für den Aufsatz und die Schraube, ohne das Werkzeug, Schutz begehrt.

Im Rahmen dieser Beschreibung ist das Werkzeug gemeinsam mit dem Aufsatz und der Schraube beschrieben. Jedoch wird auch für das Werkzeug alleine, ohne den Aufsatz und die Schraube, Schutz begehrt.

Es wird außerdem ein Verfahren zum Einsetzen und zum Lösen einer erfindungsgemäßen Schraube und eines erfindungsgemäßen Aufsatzes in einen und aus einem Wirbelkörper, beispielsweise im Rahmen einer Wirbelsäulenoperation, beschrieben. Bei diesem Verfahren handelt es sich nicht um einen Teil der Erfindung. Es dient lediglich dem Verständnis der Erfindung. J Dazu kann zuerst ein Führungsdraht gesetzt werden. Dieser kann das Setzen einer kanülierten Schraube in der richtigen Lage im Wirbelkörper vereinfachen. Die Schraube kann aber auch ohne den Führungsdraht in den Wirbelkörper eingeschraubt werden. Ein Schraubendreher kann mit der Aussparung des Schraubenkopfes gekoppelt werden und die Schraube kann in den Pedikel eingeschraubt werden. Wird eine kanülierte und gefensterte Schraube verwendet, so kann die Befestigung der Schraube in dem Knochen durch Einspritzen eines geeigneten Werkstoffs in die Kanüle der Schraube, der dann durch die Fenster der Schraube austritt und sich mit dem Knochen verbindet, insbesondere bei einem osteoporösen Knochen, verbessert werden. Dann kann das Werkzeug mit der Arretierungsvorrichtung drehfest an die Tulpe gekoppelt werden. Die Arretierung kann beispielsweise dadurch erfolgen, daß zwei an dem ersten Haltegriffendbereichs der sich diametral genüberliegende Vorsprünge mit zwei in dem Randbereich der zweiten Einführöffnung ausgebildeten, sich diametral gegenüberliegenden U-förmigen Einbuchtungen in Eingriff gebracht werden. Dabei kann das Kopplungsteil in der ersten Lage gehalten werden. Das Werkzeug kann an seinem Haltegriff gehalten werden. Das Kopplungsteil kann durch die zweite Einführöffnung der Tulpe in Richtung der zweiten Endlage geschoben werden, bis das Kopplungsteil an den Kopplungsbereich des Sattels anstößt. Dann kann der Drehgriff des Kopplungsteils gedreht werden, so daß das Außengewinde des Kopplungsteils in das Innengewinde des Sattels eingeschraubt werden kann. Die Drehbewegung des Kopplungsteils kann dadurch, wie oben beschrieben wurde, in eine Axialbewegung des Sattels übertragen werden. Somit kann der Sattel in die Freigabelage bewegt werden. Der Sattel kann mit dem Werkzeug in der Freigabelage fixiert werden. Der Aufsatz kann dann mit der ersten Einführöffnung auf den Schraubenkopf der in den Pedikel eingeschraubten Schraube zubewegt werden. Durch diese Bewegung kann der Schraubenkopf in Richtung des Sattels geführt werden. Da der Sattel in der Freigabelage fixiert ist, kann der Schraubenkopf ohne Druck in den Aufnahmebereich eingesetzt werden. Dann kann das Kopplungsteil durch Drehung an dem Drehgriff aus dem Innengewinde des Sattels herausgeschraubt werden. Dadurch kann der Sattel in die Sperrlage bewegt werden. Diese Art des Einsetzens erzeugt nur einen geringen Druck auf den Wirbel und ist insbesondere bei osteoporösen Knochen vorteilhaft. Die Tulpe ist dann polyaxial mit der Schraube verbunden.

Der Aufsatz kann aber auch ohne das Werkzeug auf den Schraubenkopf aufgesetzt werden. Dazu kann der Aufsatz derart auf die Schraube zugeführt werden, daß der Schraubenkopf in die erste Einführöffnung der Tulpe eintritt. Der Aufsatz kann weiter auf die Schraube zu bewegt werden. Dadurch kann der Kopf der Schraube gegen die Aufnahmeöffnung des Sattels stoßen. Der Sattel kann somit von der Sperrlage in die Freigabelage geschoben werden. Fällt die Freigabelage mit der ersten Lage zusammen, so kann der Sattel nicht weiter in der Einführrichtung bewegt werden. Wird der Aufsatz weiter in Richtung der Schraube bewegt, so übt der Schraubenkopf Druck die Aufnahmeöffnung auf. Der Aufnahmebereich kann sich dadurch in radialer Richtung aufweiten. Der Schraubenkopf kann durch die geweitete Aufnahmeöffnung in den Aufnahmebereich gelangen. Ist die Verformung des Aufnahmebereichs elastisch, so kann sich die Aufnahmeöffnung dann wieder verkleinern. Der Schraubenkopf ist in den Aufnahmebereich eingeschnappt, so daß die Schraube fest mit dem Aufsatz verbunden ist.

Bei dem Verfahren zum Einsetzen der erfindungsgemäßen Schraube und des erfindungsgemäßen Schraubenkopfes tritt im Vergleich zu herkömmlichen Verfahren nicht das Problem auf, daß ein polyaxial mit der Schraube verbundener Aufsatz das Einschrauben der Schraube in den Pedikel behindert.

Der Aufsatz kann mittels des Werkzeugs einfach von der Schraube gelöst werden. Dazu kann das Werkzeug am Haltegriff gehalten werden und das Kopplungsteil in die erste Endlage bewegt werden. Der erste Endbereich des Kopplungsteils mit dem Außengewinde kann in der ersten Endlage vollständig im Innenraum des Haltegriffs angeordnet sein. Das Werkzeug kann dann mit der Arretierungsvorrichtung drehfest an den Sattel gekoppelt werden. Die Arretierung kann beispielsweise dadurch erfolgen, daß zwei an dem ersten Haltegriffendbereichs angeordnete, sich diametral genüberliegende Vorsprünge mit zwei in dem Randbereich der zweiten Einführöffnung ausgebildeten, sich diametral gegenüberliegenden U-förmigen Einbuchtungen in Eingriff gebracht werden. Danach kann der erste Endbereich des Kopplungsteils mit dem Außengewinde durch die zweite Einführöffnung in den Aufsatz eingeführt werden und an dem Kopplungsbereich des Sattels herangeführt werden. Dann kann das Kopplungsteil mittels des Drehgriffs gedreht und somit das Kopplungsteil in den Kopplungsbereich des Sattels eingeschraubt werden. Durch diese Drehbewegung kann der Sattel in axialer Richtung in die Freigabelage bewegt werden. Der Aufsatz kann nun ohne großen Kraftaufwand von dem Schraubenkopf gelöst werden, indem das Werkzeug mit dem daran gekoppelten Sattel von der Schraube weg bewegt wird.

Durch dieses Verfahren kann der Aufsatz einfach, schnell und ohne großen Kraftaufwand von der Schraube gelöst werden. Dies ermöglicht ein einfaches Entfernen des Aufsatzes aus dem Körper, ohne die Schraube aus dem Knochen lösen zu müssen.

Ein erfindungsgemäßer Instrumentensatz kann zur Stabilisierung der Wirbelsäule verwendet werden. Dabei können zunächst erfindungsgemäße Schrauben gemäß dem oben beschriebenen Verfahren in die jeweils beiden Pedikel der zu verbindenden Wirbelkörper eingeschraubt werden. Danach kann, wie oben beschrieben ist, je ein erfindungsgemäßer Aufsatz auf je eine Schraube aufgesetzt werden. Das Aufsetzen kann dabei mit Hilfe des oder ohne das Werkzeug erfolgen. Danach kann je ein Stab mit den längs der Wirbelsäule angeordneten Aufsätzen verbunden werden. Da die Aufsätze polyaxial mit den Schrauben verbunden sind, ist das Einsetzen der Stäbe einfacher als in dem Fall, in dem die Aufsätze starr mit den Schrauben verbunden sind. Die Lage der Wirbelkörper kann aufgrund der polyaxialen Verbindung des Aufsatzes und der Schraube dann nochmals nachkorrigiert werden. Schließlich können die beiden Stäbe mittels der Arretierungsvorrichtung in der vorgegebenen Lage arretiert werden. Die Wirbelkörper sind dann in einer vorgegebenen Lage fixiert.

Die Schraube, die Tulpe und der Sattel können den gleichen Werkstoff, beispielsweise Ti-6Al-4V, aufweisen. Sie können aber auch verschiedene Werkstoffe aufweisen. Der Werkstoff kann einen rostfreien Stahl, beispielsweise Co-28Cr-6Mo oder X2CrNiMo18-15-3 (1.4441), aufweisen. Er kann aber auch einen anderen Werkstoff aufweisen.

In der folgenden Beschreibung wird die Erfindung unter Bezugnahme auf die Zeichnungen beispielhaft erläutert. In der Zeichnung zeigt
- Fig. 1a: eine Aufsicht auf einen Aufsatz/eine Tulpe eines Instrumentensatzes gemäß einer Ausführungsform der Erfindung mit zwei sich diametral gegenüberliegenden Einbuchtungen,
- Fig. 1b: eine Aufsicht auf den/die um 90° um die Längsachse L gedrehte/n Aufsatz/Tulpe aus Fig. 1a,
- Fig. 2: einen Schnitt der in Fig. 1a und 1b dargestellten Tulpe längs der Linie B-B (siehe Fig. 1b),
- Fig. 3: eine Aufsicht auf einen Sattel eines Instrumentensatzes gemäß einer Ausführungsform der Erfindung,
- Fig. 4: das Einsetzen des in Fig. 3 dargestellten Sattels in die in Fig. 1a, 1b und 2 dargestellte Tulpe,
- Fig. 5: auf der linken Seite einen Schnitt des und auf der rechten Seite eine Aufsicht auf den in Fig. 1a und 1b dargestellten Aufsatz/es längs der Linie B-B mit einer Tulpe und einem Sattel gemäß einer Ausführungsform der Erfindung,
- Fig. 6a: eine Aufsicht auf ein Werkzeug eines Instrumentensatzes gemäß einer Ausführungsform der Erfindung,
- Fig. 6b: eine Aufsicht auf das Kopplungsteil des in Fig. 6a dargestellten Werkzeuges,
- Fig. 6c: eine Aufsicht auf den Haltegriff des in Fig. 6a dargestellten Werkzeuges,
- Fig. 7: eine Aufsicht auf eine Schraube eines Instrumentensatzes gemäß einer Ausführungsform der Erfindung,
- Fig. 8: einen Schnitt längs der Linie B-B des in Fig. 1a und 1b dargestellten Aufsatzes, der mit der in Fig. 7 dargestellten Schraube verbunden ist,
- Fig. 9 a-d: einen Schnitt längs der Linie B-B des in Fig. 1a und 1b dargestellten Aufsatzes, der mittels des in Fig. 6a bis 6c dargestellten Werkzeugs von der in Fig. 7 dargestellten Schraube getrennt wird.

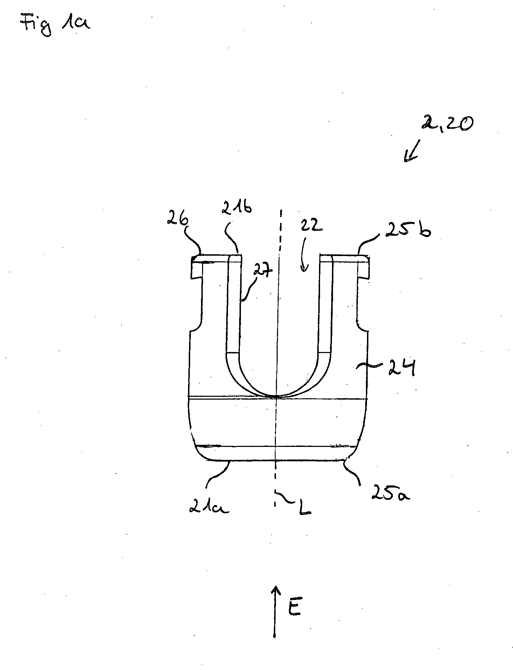

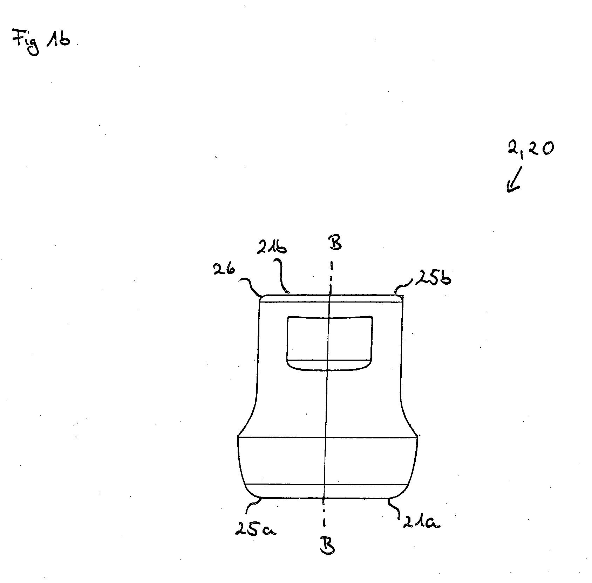

In Fig. 1a ist eine Aufsicht auf einen eine Tulpe 20 aufweisenden Aufsatz 2 eines Instrumentensatzes 1 dargestellt. Der Aufsatz 2 ist hülsenförmig und weist eine Längsachse L auf. Fig. 1b zeigt den in Fig. 1a dargestellten, die Tulpe 20 aufweisenden Aufsatz 2, der im Vergleich zu Fig. 1a um 90° um die Längsachse L gedreht ist. Die Tulpe 20 weist an einem ersten Endbereich 25a eine erste Einführöffnung 21a auf, in die ein Schraubenkopf 12 in einer Einführrichtung E in den Innenbereich 22 der Tulpe 20 eingeführt werden kann. An einem der ersten Einführöffnung 21a entgegengesetzten zweiten Endbereich 25b weist die Tulpe 20 eine zweite Einführöffnung 21b zum Einführen des Werkzeugs 40 auf. Der Randbereich 26 der zweiten Einführöffnung 21b weist zwei Einbuchtungen 27 auf. Die Einbuchtungen 27 sind sich diametral gegenüberliegend angeordnet. Die Einbuchtungen 27 sind U-förmig ausgebildet, wobei die Schenkel des U sich von der zweiten Einführöffnung 21b im Wesentlichen parallel zur Längsachse L durch die Wand 24 der Tulpe 20 erstrecken und dann durch den bogenförmigen Abschnitt des U miteinander verbunden werden. In den Einbuchtungen 27 kann ein Stab 101 eines Stabsystems 100 angeordnet werden.

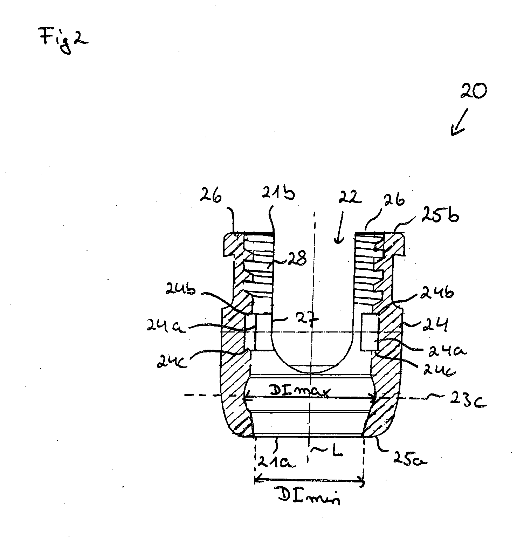

Fig. 2 zeigt einen Schnitt des in Fig. 1a und 1b dargestellten Aufsatzes 2 mit einer Tulpe 20 längs der Linie B-B (siehe Fig. 1b). Ein Innendurchmesser Dlmin der Tulpe 20 ist an der ersten Einführöffnung 21a minimal. Weiter weist die Tulpe 20 in einer Ebene 23c einen maximalen Innendurchmesser Dlmax auf. Im Bereich der Ebene 23a weist die Wand 24 der Tulpe 20 auf ihrer dem Innenraum 22 der Tulpe 20 zugewandten Seite zwei Aussparungen 24a auf, die sich diametral gegenüberliegen. Jede der Aussparungen 24a wird in axialer Richtung von einem ersten Anschlagsbereich 24b und einem zweiten Anschlagsbereich 24c begrenzt. Der erste und der zweite Anschlagsbereich 24b, c verlaufen im Wesentlichen senkrecht zur Längsachse L der Tulpe 20.

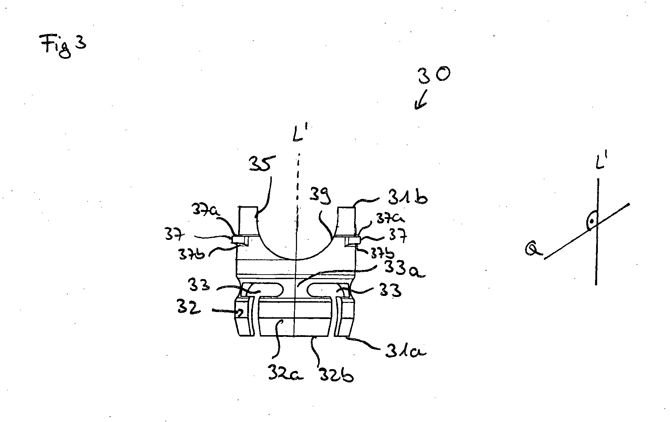

Fig. 3 zeigt eine Aufsicht auf einen Sattel 30 eines Instrumentensatzes 1 gemäß einer Ausführungsform der Erfindung. Der Sattel 30 weist an einem ersten Endbereich 31a einen Aufnahmebereich 32 zur Aufnahme des Schraubenkopfes 12 auf. Der Aufnahmebereich 32 wird durch eine Wand 32a begrenzt und weist eine Aufnahmeöffnung 32b auf, durch welche der Schraubenkopf 12 in den Aufnahmebereich 32 eingeführt werden kann. Die Wand 32a des Aufnahmebereichs 32 weist mehrere Schlitze 33 auf. Die Schlitze 33 unterteilen die Wand 32a des Aufnahmebereichs 32 in mehrere voneinander getrennte Bereiche. Die Schlitze 33 sind T-förmig ausgebildet. Dabei erstreckt sich der vertikale Balken des T jeweils im Wesentlichen senkrecht zum Rand der Aufnahmeöffnung 32b durch die Wand 32a des Aufnahmebereichs 32 und geht dann in den horizontalen Balken des T über. Dadurch weist die Wand 32a des Aufnahmebereichs 32 in einem Bereich zwischen dem ersten Endbereich 31a und einem entgegengesetzt dem ersten Endbereich 31a angeordneten zweiten Endbereich 31b zwischen zwei benachbarten T-förmigen Schlitzen 33 nur eine schmale Werkstoffbrücke 33a auf. Dies ermöglicht eine reversible Verformung der Aufnahmeöffnung 32b und des Aufnahmebereichs 32 in einer senkrecht zu einer Längsachse L der Tulpe 20 verlaufenden Querrichtung Q. Der zweite Endbereich 31b weist einen Kopplungsbereich 35 zur Kopplung mit dem Werkzeug 40 auf. Weiter weist der Sattel 30 an dem zweiten Endbereich 31b eine sich diametral durch den Sattel 30 erstreckende Aussparung 39 auf. In der Aussparung 39 kann der in den Einbuchtungen 27 des Randbereichs 26 der Tulpe 20 anordenbare Stab 101 aufgenommen werden. Die Aussparung 39 ist U-förmig ausgebildet. Dabei erstrecken sich die Schenkel des U im Wesentlichen parallel zur Längsachse L' des Sattels 30 und werden dann durch den bogenförmigen Abschnitt des U miteinander verbunden. Zwischen dem ersten Endbereich 31a und dem zweiten Endbereich 31b des Sattels 30 weist der Sattel 30 zwei sich diametral gegenüberliegend angeordnete Vorsprünge 37 auf. Die Vorsprünge 37 weisen jeweils eine Komponente senkrecht zur Längsachse L' des Sattels 30 auf. Wie später erklärt wird, können die Vorsprünge 37 in die Aussparungen 24a der Tulpe 20 eingreifen und so die axiale Bewegung des Sattels 30 in der Tulpe 20 zwischen einer ersten Lage L1 und einer zweiten Lage L2 begrenzen.

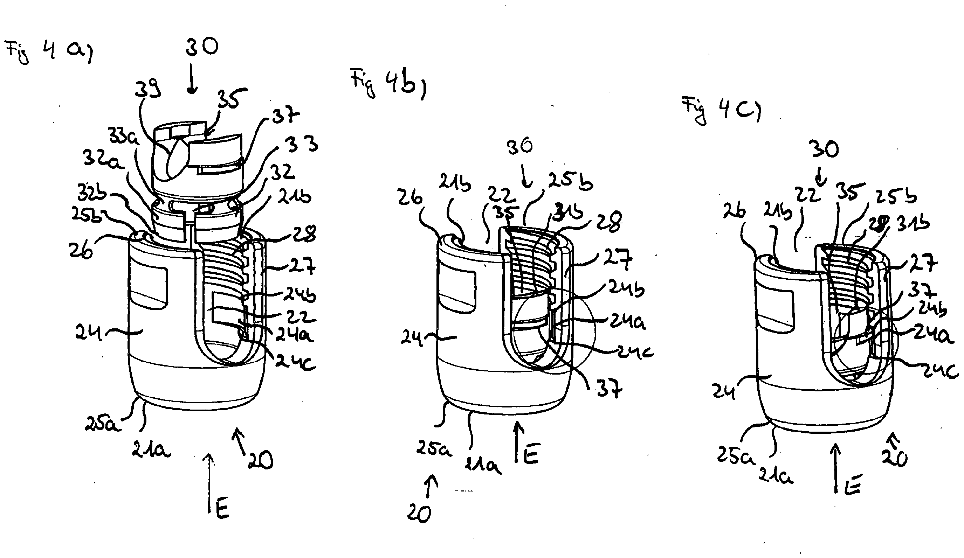

Fig. 4a bis g zeigen das Einsetzen des in Fig. 3 dargestellten Sattels 30 in die in Fig. 1a, 1b und 2 dargestellte Tulpe 20. Wie in Fig. 4a dargestellt ist, wird der Sattel 30 mit dem Aufnahmebereich 32 voraus durch die zweite Einführöffnung 21b in die Tulpe 20 eingesetzt. Dabei ist der Sattel 30 gegenüber der Tulpe 20 derart ausgerichtet, daß die beiden sich diametral gegenüberliegenden Vorsprünge 37 des Sattels 30 mit den U-förmigen Einbuchtungen 27 der Tulpe 20 zusammenfallen.

Wie in Fig. 4b dargestellt ist, wird der Sattel 30 so weit in die Tulpe 20 eingeführt, daß die Vorsprünge 37 auf der Höhe der in der Wand 24 der Tulpe 20 angebrachten Aussparungen 24a angeordnet sind.

Wie in Fig. 4c gezeigt ist, wird der Sattel 30 dann gegenüber der Tulpe 20 derart verdreht, daß die Vorsprünge 37 in die Aussparungen 24a der Tulpe 20 eingeführt werden. Der Sattel 30 kann auf diese Weise drehfest mit der Tulpe 20 verbunden werden.

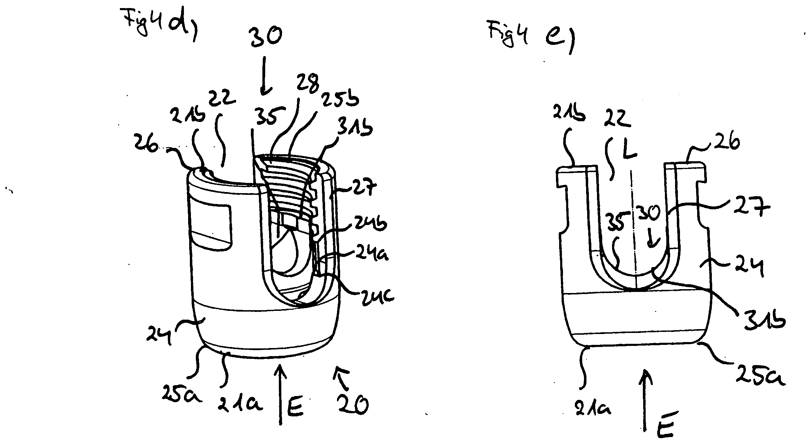

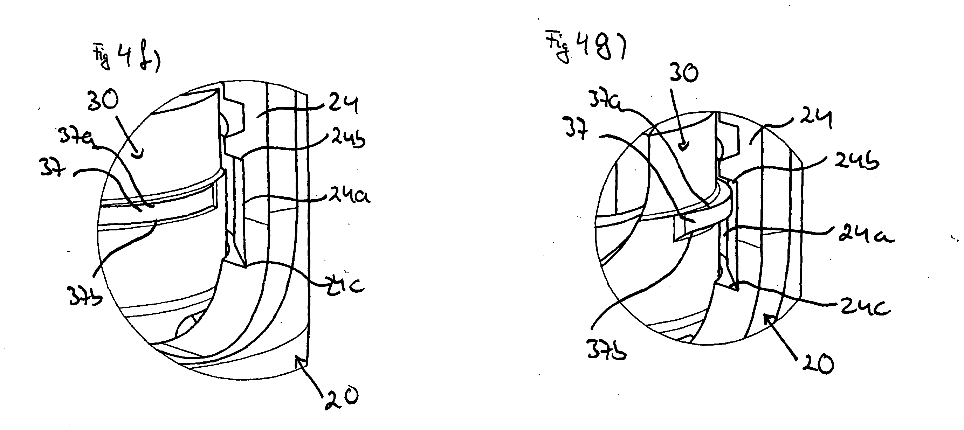

Fig. 4d zeigt den drehfest in der Tulpe 20 angeordneten Sattel 30. Fig. 4e zeigt die in Fig. 1a dargestellte Aufsicht auf die Tulpe 20, in die der Sattel 30 eingesetzt ist. Die Längsachse L der Tulpe 20 fällt im wesentlichen mit der Längsachse L' des Sattels 30 zusammen. Fig. 4f stellt eine Vergrößerung des in Fig. 4b kreisförmig markierten Ausschnitts dar. Der Sattel 30 ist noch nicht drehfest mit der Tulpe 20 verbunden. Fig. 4g zeigt eine Vergrößerung des in Fig. 4c kreisförmig markierten Ausschnitts. Der Sattel 30 wird derart gegenüber der Tulpe 20 verdreht, daß der Vorsprung 37 in die Aussparung 24a der Tulpe 20 eingeführt wird und der Sattel 30 drehfest mit der Tulpe 20 verbunden wird.

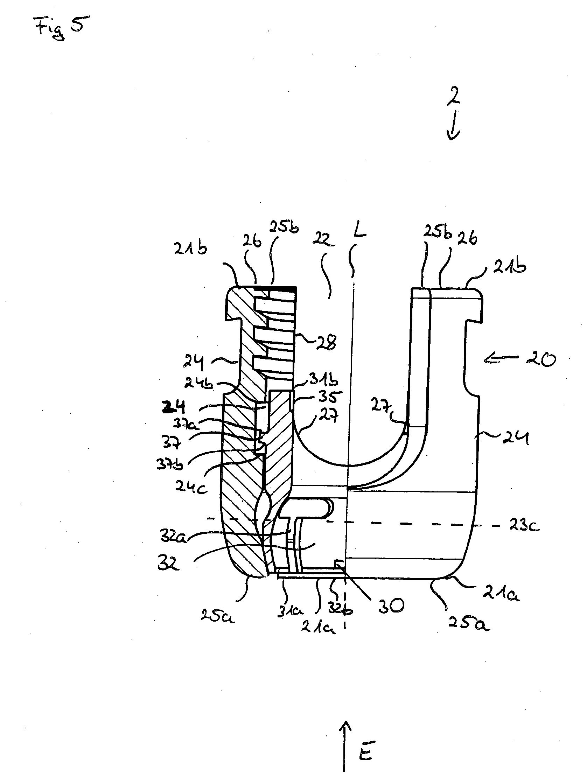

Fig. 5 zeigt den in Fig. 1a dargestellten Aufsatz 2. Die rechte Seite von Fig. 5 zeigt dabei eine Aufsicht des Aufsatzes 2. Die linke Seite von Fig. 5 zeigt einen Schnitt durch den Aufsatz 2 entlang der Linie B-B (siehe Fig. 1b). Der Sattel 30 ist in die Tulpe 20 eingesetzt. Die Aufnahmeöffnung 32b des Sattels 30 und die erste Einführöffnung 21a der Tulpe 20 liegen annähernd in derselben Ebene. Der Sattel 30 ist in der Sperrlage 23b angeordnet. Aus der Figur ist ersichtlich, daß der maximale Durchmesser der Aufnahmeöffnung 32b durch den minimalen Innendurchmesser Dlmin der Tulpe 20 an der ersten Einführöffnung 21a auf eine Sperrausdehnung DS begrenzt ist. Wird der Sattel 30 in der Tulpe 20 in der Einführrichtung E in axialer Richtung bewegt, so daß die Aufnahmeöffnung 32b in der Ebene 23c angeordnet ist, so ist der Sattel 30 in der Freigabelage 23a angeordnet. Nun kann sich die Aufnahmeöffnung 32b auf die Freigabeausdehnung DF ausdehnen, die größer als der maximale Durchmesser des Schraubenkopfes 12 ist, so daß der Schraubenkopf 12 in den Aufnahmebereich 32 eingeführt und aus dem Aufnahmebereich 32 gelöst werden kann. Der Sattel 30 weist einen sich in Richtung der Wand 24 der Tulpe 20 erstreckenden Vorsprung 37 auf. Der Vorsprung 37 weist eine erste Anschlagfläche 37a und eine zweite Anschlagfläche 37b auf. Die erste Anschlagfläche 37a und die zweite Anschlagfläche 37b weisen eine Komponente senkrecht zur Längsachse L der Tulpe 20 auf. Wird der Sattel 30 in der Tulpe 20 in der Einführrichtung E verschoben, so stößt die erste Anschlagfläche 37a des Vorsprungs 37 gegen den ersten Anschlagsbereich 24b der Aussparung 24a an. Dadurch wird die erste Lage L1 des Sattels 30 definiert. Der Sattel 30 kann nicht über die erste Lage L1 hinaus in der Einführrichtung E verschoben werden. Wird der Sattel 30 entgegen der Einführrichtung E bewegt, so stößt die zweite Anschlagfläche 37b des Vorsprungs 37 an den zweiten Anschlagsbereich 24c an. Dies definiert die zweite Lage L2 des Sattels 30. Der Sattel 30 kann nicht über die zweite Lage L2 hinaus entgegen der Einführrichtung E bewegt werden. Die Bewegung des Sattels 30 ist also in axialer Richtung zwischen der ersten Lage L1 und der zweiten Lage L2 beschränkt. Insbesondere kann der Sattel 30 nicht aus der Tulpe 20 herausfallen. Die hülsenförmige Tulpe 20 weist an einem der ersten Einführöffnung 21a entgegengesetzt angeordneten zweiten Endbereich 25b eine zweite Einführöffnung 21b auf. Das Werkzeug 40 kann durch die zweite Einführöffnung 21b in die Tulpe 20 eingeführt werden. Das Werkzeug 40 kann dann mit dem Kopplungsbereich 35 des Sattels 30 gekoppelt werden. Der Kopplungsbereich 35 kann dazu ein Innengewinde 38 aufweisen. Der Randbereich 26 der zweiten Einführöffnung 21b weist zwei sich diametral gegenüberliegende U-förmige Einbuchtungen 27 zur Aufnahme eines Stabes 101 eines Stabsystems 100 auf. Weiter weist der dem Aufnahmebereich 32 des Sattels 30 entgegengesetzte zweite Endbereich 31b eine sich diametral durch den Sattel 30 erstreckende U-förmige Aussparung 39 zur Aufnahme des in den Einbuchtungen 27 der Tulpe 20 angeordneten Stabes 101 auf. Der Stab 101 kann dann durch eine an dem zweiten Endbereich 25b der Tulpe 20 angeordnete Arretierungsvorrichtung 28 in der Tulpe 20 fixiert werden. Wie in Fig. 5 dargestellt ist, kann die Arretierungsvorrichtung 28 als ein Innengewinde ausgebildet sein. Beispielsweise kann eine Madenschraube 50 mit dem Innengewinde verschraubt werden, wodurch der Stab 101 in dem Aufsatz 2 fixiert wird.

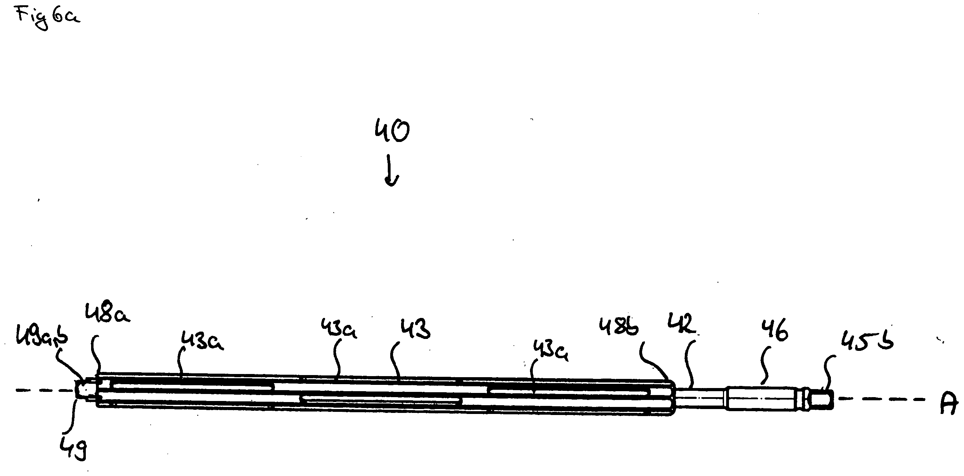

Fig. 6a zeigt eine Aufsicht auf ein Werkzeug 40 eines Instrumentensatzes 1 gemäß einer Ausführungsform der Erfindung. Das Werkzeug 40 weist einen länglichen Haltegriff 43 auf. Der Innenraum 44 des Haltegriffs 43 weist einen Hohlraum auf, in dem ein zylinderförmiges Kopplungsteil 42 angeordnet ist. Das Kopplungsteil 42 ist gegenüber dem Haltegriff 43 in axialer Richtung zwischen einer ersten Endlage 47a und einer zweiten Endlage 47b verschiebbar. In der ersten Endlage 47a ist das Kopplungsteil 42 so weit in den Innenraum 44 des Haltegriffs 43 zurückgezogen, daß der in Richtung des ersten Haltegriffendbereichs 48a angeordnete erste Endbereich 45a des Kopplungsteils 42 in dem Innenraum 44 des Haltegriffs 43 angeordnet ist. Dieser Fall ist in Fig. 6a dargestellt. In der zweiten Endlage 47b ragt der erste Endbereich 45a des Kopplungsteils 42 über den ersten Haltegriffendbereich 48a des Haltegriffs 43 hervor (nicht dargestellt). Das Kopplungsteil 42 ist um seine Längsachse A drehbar in dem Innenraum 44 des Haltegriffs 43 angeordnet. Das Werkzeug 40 kann an dem Haltegriff 43 gehalten werden, und das Kopplungsteil 42 kann über seinen Drehgriff 46 gegenüber dem Haltegriff 43 gedreht werden. Der Haltegriff 43 weist an einem dem Drehgriff 46 des Kopplungsteils 42 entgegengesetzt angeordneten ersten Haltegriffendbereich 48a eine Arretierungsvorrichtung 49 auf. Die Arretierungsvorrichtung 49 weist einen ersten Vorsprung 49a und einen zweiten Vorsprung 49b auf. Der erste Vorsprung 49a und der zweite Vorsprung 49b können mit den sich diametral gegenüberliegenden Einbuchtungen 27 im Randbereich 26 der der Tulpe 20 in Eingriff gebracht werden, wodurch das Werkzeug 40 drehfest mit der Tulpe 20 gekoppelt werden kann.

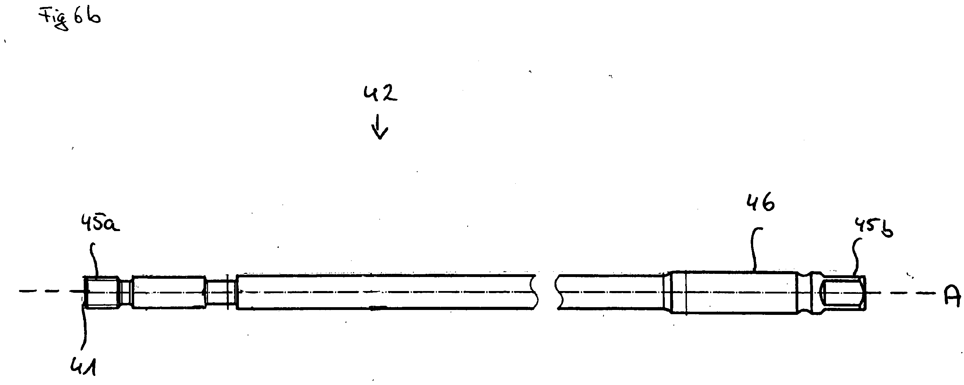

Fig. 6b zeigt eine Aufsicht auf das Kopplungsteil 42 des in Fig. 6a dargestellten Werkzeugs 40. Das Kopplungsteil 42 ist zylinderförmig ausgebildet und weist einen ersten Endbereich 45a und einen zweiten Endbereich 45b auf. An dem zweiten Endbereich 45b ist ein Drehgriff 46 angeordnet. Der erste Endbereich 45a des Kopplungsteils 42 kann an den Kopplungsbereich 35 des Sattels 30 gekoppelt werden. Der erste Endbereich 45a weist dazu ein Außengewinde 41 auf, das mit einem in dem Kopplungsbereich 35 des Sattels 30 angeordneten Innengewinde 38 verschraubt werden kann.

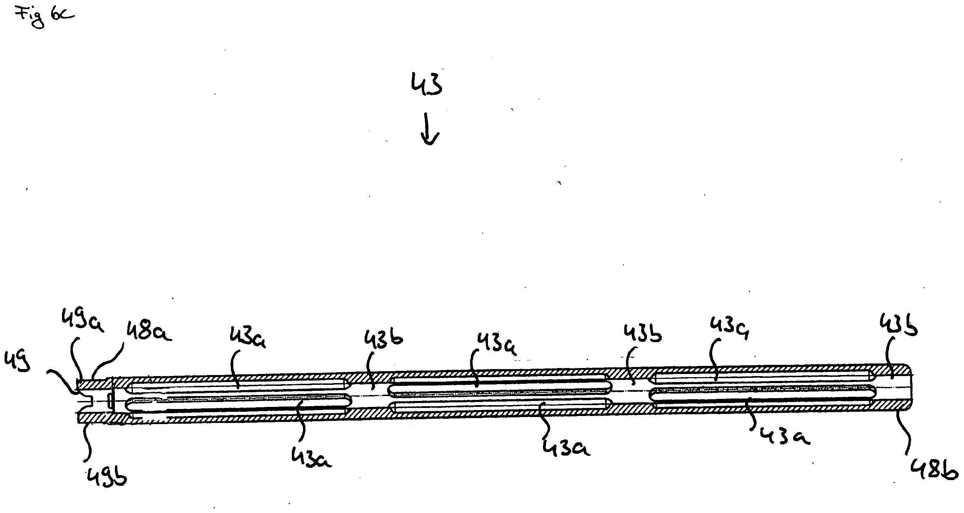

Fig. 6c zeigt eine Aufsicht auf den Haltegriff 43 des in Fig. 6a dargestellten Werkzeugs 40. Der Haltegriff 43 ist länglich ausgebildet. Die Wand des Haltegriffs 43 weist Rillen 43a und Fenster 43b auf, wodurch die Griffigkeit des Haltegriffs 43 verbessert wird. Das Werkzeug 40 weist einen ersten Haltegriffendbereich 48a und einen zweiten Haltegriffendbereich 48b auf. Der erste Haltegriffendbereich 48a ist in der Richtung des ersten Endbereichs 45a des in den Haltegriff 43 eingesetzten Kopplungsteils 42 angeordnet. Der zweite Haltegriffendbereich 48b ist an dem dem ersten Haltegriffendbereich entgegengesetzten Ende des Haltegriffs 43 angeordnet. An dem ersten Haltegriffendbereich 48a des Werkzeugs 40 ist eine Arretierungsvorrichtung 49 angeordnet. Durch die Arretierungsvorrichtung 49 kann das Werkzeug 40 drehfest mit der Tulpe 20 gekoppelt werden. Die Arretierungsvorrichtung 49 weist einen ersten Vorsprung 49a und einen zweiten Vorsprung 49b auf. Der erste Vorsprung 49a und der zweite Vorsprung 49b sind sich diametral gegenüberliegend angeordnet und derart ausgestaltet, daß sie mit den sich diametral gegenüberliegenden Einbuchtungen 27 des Randbereichs 26 der Tulpe 20 in Eingriff gebracht werden können.

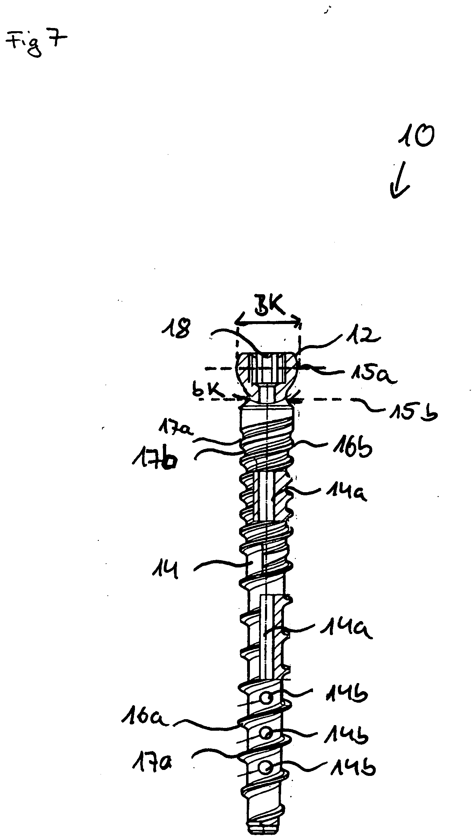

Fig. 7 zeigt eine Aufsicht auf eine Schraube 10 eines Instrumentensatzes 1 gemäß einer Ausführungsform der Erfindung. Die Schraube 10 weist einen Schaft 14 zum Einschrauben in einen Knochen auf. An dem Schaft 14 sind ein erstes Gewinde 17a und ein zweites Gewinde 17b angeordnet. Das erste Gewinde 17a und das zweite Gewinde 17b haben dieselbe Gewindesteigung. Das erste Gewinde 17a erstreckt sich entlang des gesamten Schaftes 14. Das zweite Gewinde 17b erstreckt sich nur entlang eines zweiten Schaftbereichs 16b, der an den Schraubenkopf 12 angrenzt. Die Schraube 10 weist also im zweiten Schaftbereich 16b ein zweigängiges Gewinde auf. In einem ersten Schaftbereich 16a, der an den zweiten Schaftbereich 16b angrenzt und sich von diesem entgegengesetzt zum Schraubenkopf 12 erstreckt, weist die Schraube 10 ein eingängiges Gewinde auf. Wie in Fig. 7 dargestellt ist, weist der Schaft 14 eine Kanüle 14a auf. Dadurch kann die Schraube 10 beim Einschrauben in einen Knochen über einen Führungsdraht geführt werden. Weiter weist der Schaft 14 der Schraube 10 Fenster 14b auf. Wird die Schraube 10 beispielsweise in einen osteoporösen Knochen einschraubt, so kann der Halt der Schraube 10 in dem Knochen dadurch verbessert werden, daß ein Werkstoff in die Kanüle 14a der Schraube 10 eingespritzt wird, der durch die Fenster 14b austritt und sich mit dem Knochen verbindet. An den Schaft 14 der Schraube 10 schließt sich der Schraubenkopf 12 an. Der Schraubenkopf 12 weist die Form einer Kugelscheibe auf. Der Schraubenkopf 12 weist einen ersten Kopfbereich 15a auf, der dadurch gekennzeichnet ist, daß der Schraubenkopf 12 in einer Richtung senkrecht zum Schaft 14 eine maximale Breite BK aufweist. Zwischen dem ersten Kopfbereich 15a und dem Schaft 14 ist ein zweiter Kopfbereich 15b angeordnet, der in der Richtung senkrecht zum Schaft 14 eine zweite Breite bK aufweist, die kleiner als die maximale Breite BK ist. Durch diese Ausgestaltung des Schraubenkopfes 12 kann der Schraubenkopf 12 in der Sperrlage 23b des Sattels 30 untrennbar mit dem Aufnahmebereich 32 des Sattels 30 verbunden werden.

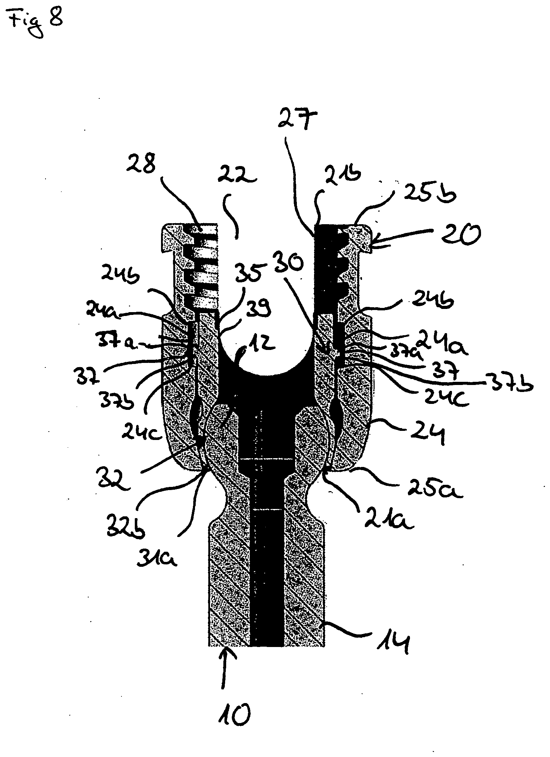

Fig. 8 zeigt einen Schnitt entlang der Linie B-B des in Fig. 1a und 1b dargestellten Aufsatzes 2, der mit der in Fig. 7 dargestellten Schraube 10 verbunden ist. Der Schraubenkopf 12 ist in dem Aufnahmebereich 32 des Sattels 30 angeordnet. Der Sattel 30 ist in der Sperrlage 23b in der Tulpe 20 angeordnet. Die maximale Ausdehnung der Aufnahmeöffnung 32b ist hier auf eine Sperrausdehnung DS begrenzt. Die Sperrausdehnung DS ist kleiner als die maximale Breite BK des Schraubenkopfes 12. Werden die Schraube 10 und der Aufsatz 2 in entgegengesetzter Richtung voneinander weg bewegt, so kann der Sattel 30 aufgrund des an dem Sattel 30 angeordneten Vorsprungs 37, der in die in der Wand 24 der Tulpe 20 ausgebildete Aussparung 24a eingreift, nicht über die zweite Lage L2 hinausbewegt werden. Die maximale Ausdehnung der Aufnahmeöffnung 32b ist auch in der zweiten Lage L2 auf die Sperrausdehnung DS begrenzt. Dadurch ist die Schraube 10 unlösbar mit dem Aufsatz 2 verbunden.

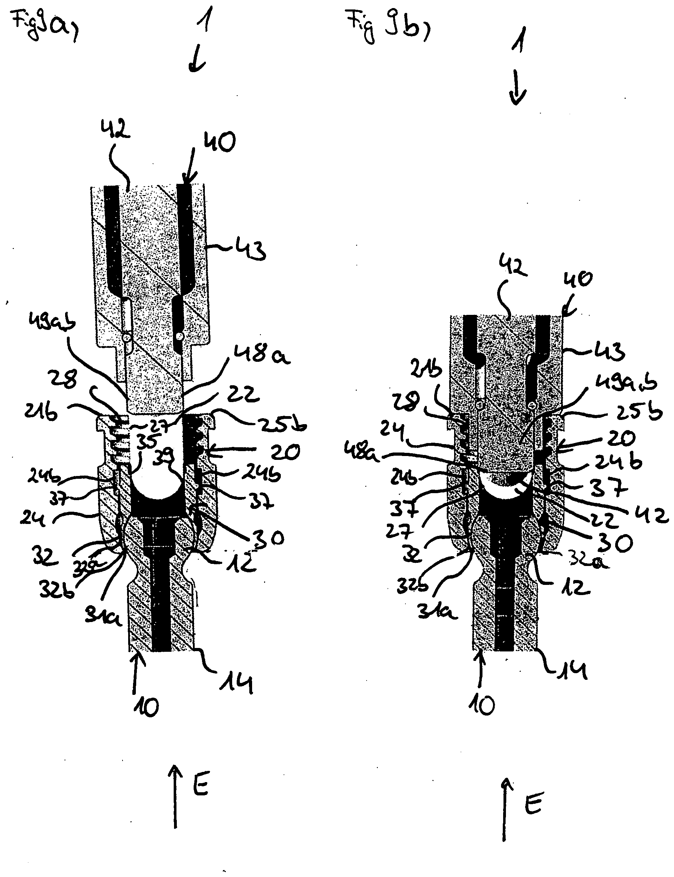

Fig. 9a bis d zeigen den Schnitt entlang der Linie B-B des in Fig. 1a und 1b dargestellten Aufsatzes 2, der mittels des in Fig. 6a bis 6c dargestellten Werkzeuges 40 von der in Fig. 7 dargestellten Schraube 10 getrennt wird. Fig. 9a zeigt den mit der Schraube 10 verbundenen Aufsatz 2. Der Schraubenkopf 12 ist in dem Aufnahmebereich 32 des Sattels angeordnet. Der Sattel 30 befindet sich in der Sperrlage 23b. Der erste Haltegriffendbereich 48a des Werkzeugs 40 wird auf die an dem zweiten Endbereich 25b angeordnete zweite Einführöffnung 21b der Tulpe 20 zugeführt. Das Kopplungsteil 42 ist in der ersten Endlage 47a im Innenraum 44 des Haltegriffs 43 des Werkzeugs 40 angeordnet.

Fig. 9b zeigt das nun drehfest mit der Tulpe 20 gekoppelte Werkzeug 40. Der an dem ersten Haltegriffendbereich 48a angeordnete erste und zweite Vorsprung 49a, b ist dazu mit den beiden sich diametral gegenüberliegenden Einbuchtungen 27 des Randbereichs 26 der zweiten Einführöffnung 21b in Eingriff gebracht worden. Der mit dem Schraubenkopf 12 verbundene Sattel 30 ist in der Sperrlage 23b angeordnet. Das Kopplungsteil 42 ist von der ersten Endlage 47a entgegen der Einführrichtung in Richtung der zweiten Endlage 47b bewegt worden und ragt nun etwas über den ersten Haltegriffendbereich 48a hervor. Das Kopplungsteil 42 wird so weit entgegen der Einführrichtung E auf den Sattel 30 zu bewegt, bis das Außengewinde 41 des Kopplungsteils 42 den Kopplungsbereich 35 des Sattels 30 berührt und mit dem Innengewinde 38 des Kopplungsbereichs 35 des Sattels 30 verschraubt werden kann.

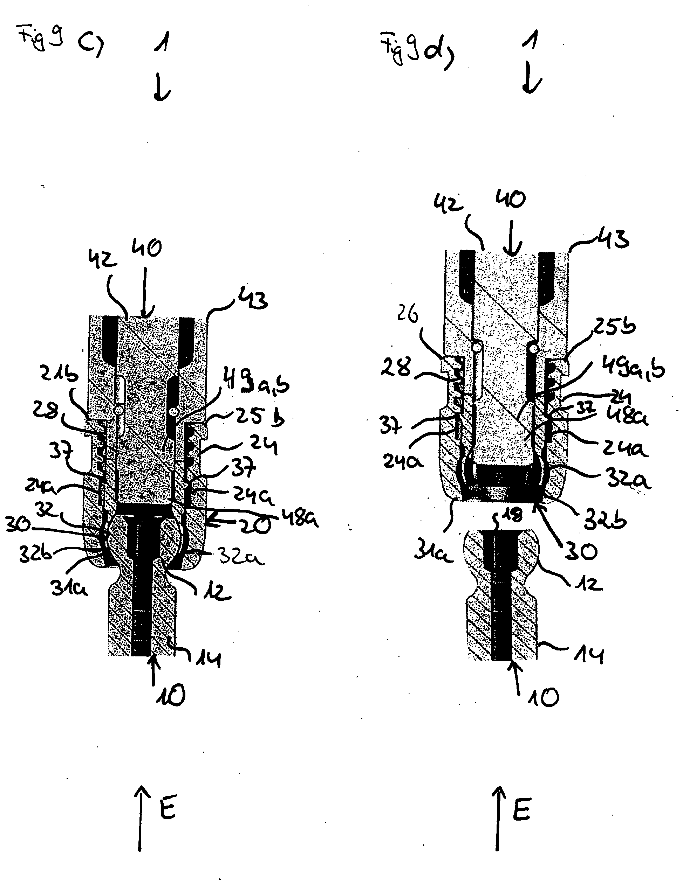

Fig. 9c zeigt das drehfest mit der Tulpe 20 gekoppelte Werkzeug 40. Die Schraube 10 ist über den in dem Aufnahmebereich 32 des Sattels angeordneten Schraubenkopf 12 mit der Tulpe 20 verbunden. Durch die drehfeste Verbindung des Werkzeugs 40 mit der Tulpe 20 wird die Drehbewegung des Kopplungsteils 42 beim Einschrauben in das Innengewinde 38 des Sattels 30 in eine Axialbewegung des Sattels 30 in der Einführrichtung E übertragen.

Dadurch wird, wie in Fig. 9c dargestellt ist, der Sattel 30 aus der Sperrlage 23b in der Einführrichtung E in die Freigabelage 23a bewegt. In der Freigabelage 23a des Sattels 30 weist die Tulpe 20 in einer mit der Aufnahmeöffnung 32b des Sattels 30 zusammenfallenden Ebene 23c einen maximalen Innendurchmesser Dlmax auf. Der maximale Innendurchmesser Dlmax der Tulpe 20 in der durch die Freigabelage 23a definierten Ebene 23c erlaubt eine maximale Ausdehnung der Aufnahmeöffnung 32b des Aufnahmebereichs 32 des Sattels 30 in einer Querrichtung Q auf eine Freigabeausdehnung DF, die größer ist als die maximale Breite BK des Schraubenkopfes 12.

Werden nun, wie in Fig. 9d dargestellt ist, die Schraube 10 und das Werkzeug 40 voneinander weg bewegt, so kann der Schraubenkopf 12 aus dem Aufnahmebereich 32 des Sattels 30 gelöst werden. Dies ist möglich, da das Werkzeug 40 die Lage des Sattels 30 in der Freigabelage 23a fixiert.

Werden die in Fig. 9a bis 9d dargestellten Schritte in umgekehrter Reihenfolge ausgeführt, so kann die Schraube 10 mit einem kleinen Kraftaufwand in axialer Richtung mit dem Aufsatz 2 verbunden werden.

### Verzeichnis der Bezuqszeichen

- 1: Instrumentensatz
- 2: Aufsatz
- 10: Schraube
- 12: Schraubenkopf
- 14: Schaft
- 14a: Kanüle
- 14b: Fenster
- 15a: erster Kopfbereich
- 15b: zweiter Kopfbereich
- 16a: erster Schaftbereich
- 16b: zweiter Schaftbereich
- 17a: erstes Gewinde
- 17b: zweites Gewinde
- 18: Aussparung
- BK: maximale Breite des Schraubenkopfes
- bK: zweite Breite des Schraubenkopfes
- 20: Tulpe
- 21a: erste Einführöffnung
- 21b: zweite Einführöffnung
- 22: Innenbereich
- 23a: Freigabelage
- 23b: Sperrlage
- 23c: Ebene
- 24: Wand
- 24a: Aussparung
- 24b: erster Anschlagsbereich
- 24c: zweiter Anschlagsbereich
- 25a: erster Endbereich
- 25b: zweiter Endbereich
- 26: Randbereich
- 27: Einbuchtung
- 28: Arretierungsvorrichtung
- Dlmin: minimaler Innendurchmesser
- Dlmax: maximaler Innendurchmesser
- 30: Sattel
- 31a: erster Endbereich
- 31b: zweiter Endbereich
- 32: Aufnahmebereich
- 32a: Wand
- 32b: Aufnahmeöffnung
- 33: Schlitz
- 33a: Werkstoffbrücke
- 35: Kopplungsbereich
- 37: Vorsprung
- 37a: erste Anschlagfläche
- 37b: zweite Anschlagfläche
- 38: Innengewinde
- 39: Aussparung
- L1: erste Lage
- L2: zweite Lage
- DS: Sperrausdehnung
- DF: Freigabeausdehnung
- 40: Werkzeug
- 41: Außengewinde
- 42: Kopplungsteil
- 43: Haltegriff
- 43a: Fenster
- 43b: Rillen
- 44: Innenraum
- 45a: erster Endbereich
- 45b: zweiter Endbereich
- 46: Drehgriff
- 47a: erste Endlage
- 47b: zweite Endlage
- 48a: erster Haltegriffendbereich
- 48b: zweiter Haltegriffendbereich
- 49: Arretierungsvorrichtung
- 49a: erster Vorsprung
- 49b: zweiter Vorsprung
- 50: Madenschraube
- 60: Schraubenzieher
- 100: Stabsystem
- 101: Stab
- E: Einführrichtung
- Q: Querrichtung
- L: Längsachse der Tulpe
- L': Längsachse des Sattels
- A: Längsachse des Kopplungsteils

## Patentansprüche

1. Instrumentensatz (1) zum Verbinden von Wirbelkörpern mit einer in einen Pedikel einschraubbaren, einen Schraubenkopf (12) und einen Schaft (14) aufweisenden Schraube (10), einem mit der Schraube (10) polyaxial verbindbaren Aufsatz (2) zur Kopplung an ein Stabsystem (100) mit einer hülsenförmigen Tulpe (20) mit einer ersten Einführöffnung (21a) zum Einführen des Schraubenkopfes (12) in einer Einführrichtung (E) und einem Werkzeug (40), wobei der Aufsatz (2) im Innenbereich (22) der Tulpe (20) einen Sattel (30) aufweist, der Sattel (30) drehfest in der Tulpe (20) anordenbar ist und an einem der ersten Einführöffnung (21a) zugewandten ersten Endbereich (31a) einen Aufnahmebereich (32) zum Aufnehmen des Schraubenkopfes (12) und an einem dem Aufnahmebereich (32) entgegengesetzten zweiten Endbereich (31b) einen mit dem Werkzeug (40) koppelbaren Kopplungsbereich (35) aufweist und zwischen einer durch Einführen des Schraubenkopfes (12) erreichbaren Freigabelage (23a) und einer durch Verlagern des Schraubenkopfes (12) aus der Freigabelage (23a) entgegen der Einführrichtung (E) erreichbaren Sperrlage (23b) beweglich ist, wobei der in dem Aufnahmebereich (32) aufgenommene Schraubenkopf (12) in der Sperrlage (23b) nicht aus dem Aufnahmebereich (32) lösbar ist, und der Sattel (30) durch das an den Kopplungsbereich (35) gekoppelte Werkzeug (40) in einer das Freigeben des Schraubenkopfes (12) aus dem Aufnahmebereich (32) ermöglichenden Freigabelage (23a) fixierbar ist, so dass die Schraube (10) und der Aufsatz (2) voneinander trennbar sind, **dadurch gekennzeichnet, daß** der Kopplungsbereich (35) des Sattels (30) ein Innengewinde (38) aufweist, das Werkzeug einen Haltegriff (43) mit einem hohlen Innenraum (44) und ein Kopplungsteil (42) zur Kopplung an den Kopplungsbereich (35) des Sattels (30) aufweist, wobei das Kopplungsteil (42) zumindest teilweise in dem Innenraum (44) angeordnet ist und um eine Längsachse (A) drehbar ist, das Kopplungsteil (42) des Werkzeugs (40) an einem erstem Endbereich (45a) ein Außengewinde (41) zum Verschrauben mit dem Innengewinde (38) des Kopplungsbereichs (35) aufweist und das Werkzeug (40) eine Arretierungsvorrichtung (49) aufweist, mit der der Haltegriff (43) drehfest mit der Tulpe (20) verbindbar ist.

2. Instrumentensatz nach Anspruch 1, **dadurch gekennzeichnet, daß** der durch eine Wand (32a) begrenzte und eine Aufnahmeöffnung (32b) zum Einführen des Schraubenkopfes (12) aufweisende Aufnahmebereich (32) in einer senkrecht zu einer Längsachse (L) der Tulpe (20) verlaufenden Querrichtung (Q) reversibel verformbar ist.

3. Instrumentensatz nach Anspruch 2, **dadurch gekennzeichnet, daß** der Aufnahmebereich (32) elastisch verformbar ist.

4. Instrumentensatz nach Anspruch 2 oder 3, **dadurch gekennzeichnet, daß** die Wand (32a) des Aufnahmebereichs (32) durch mindestens zwei Schlitze und/oder Fenster (33) in mindestens zwei voneinander getrennte Bereiche unterteilt ist.

5. Instrumentensatz nach Anspruch 4, **dadurch gekennzeichnet, daß** die Schlitze und/oder Fenster (33) T-förmig ausgebildet sind.

6. Instrumentensatz nach Anspruch 2 bis 5, **dadurch gekennzeichnet, daß** zumindest in der Sperrlage (23b) eine Wand (24) der Tulpe (20) eine Verformung des Aufnahmebereichs (32) in der Querrichtung (Q) begrenzt.

7. Instrumentensatz nach einem der vorangehenden Ansprüche, **dadurch gekennzeichnet, daß** der Schraubenkopf (12) in einem ersten Kopfbereich (15a) in einer Richtung senkrecht zum Schaft (14) eine maximale Breite (BK) aufweist, und ein zwischen dem ersten Kopfbereich (15a) und dem Schaft (14) angeordneter zweiter Kopfbereich (15b) in der Richtung senkrecht zum Schaft (14) eine zweite Breite (bK) aufweist, die kleiner als die maximale Breite (BK) ist.

8. Instrumentensatz nach Anspruch 6 bis 7, **dadurch gekennzeichnet, daß** in der Sperrlage (23b) des Sattels (30) die Wand (24) der Tulpe (20) eine durch eine Verformung in Querrichtung (Q) erreichbare maximale Ausdehnung der Aufnahmeöffnung (32b) auf eine Sperrausdehnung (DS) begrenzt, die kleiner als die maximale Breite (BK) und größer oder gleich der zweiten Breite (bK) des Schraubenkopfes (12) ist, und in der Freigabelage (23a) des Sattels (30) die Wand der Tulpe (20) eine durch Verformung in Querrichtung (Q) erreichbare maximale Ausdehnung der Aufnahmeöffnung (32b) auf eine Freigabeausdehnung (DF) erlaubt, die größer ist als die maximale Breite (BK) des Schraubenkopfes (12).

9. Instrumentensatz nach einem der vorangehenden Ansprüche, **dadurch gekennzeichnet, daß** ein Innendurchmesser (Dlmin) der Tulpe (20) an der ersten Einführöffnung (21) minimal ist und ein Innendurchmesser (Dlmax) der Tulpe (20) in einer durch die Aufnahmeöffnung (32b) des Sattels (30) in der Freigabelage (23a) definierten Ebene (23c) maximal ist.

10. Instrumentensatz nach einem der vorangehenden Ansprüche, **dadurch gekennzeichnet, daß** die Axialbewegung des Sattels (30) in der Tulpe (20) zwischen einer ersten Lage (L1) und einer zweiten Lage (L2) begrenzt ist.

11. Instrumentensatz nach Anspruch 10, **dadurch gekennzeichnet, daß** die erste Lage (L1) mit der Freigabelage (23a) zusammenfällt.

12. Instrumentensatz nach Anspruch 10 oder 11, **dadurch gekennzeichnet, daß** der Sattel (30) zwischen dem Aufnahmebereich (32) und dem Kopplungsbereich (35) mindestens einen zur Wand (24) hin hervorspringenden, in eine in der Wand (24) der Tulpe (20) ausgebildete Aussparung (24a) eingreifenden Vorsprung (37) aufweist, wobei der bei einer Axialbewegung des Sattels (30) an einen die Aussparung (24a) in axialer Richtung begrenzenden ersten und zweiten Anschlagsbereich (24 b, c) anstoßende Vorsprung (37) die Bewegung des Sattels (30) zwischen der ersten (L1) und der zweiten Lage (L2) begrenzt.

13. Instrumentensatz nach einem der vorangehenden Ansprüche, **dadurch gekennzeichnet, daß** die Tulpe (20) und der Sattel (30) miteinander lösbar verbunden sind.

14. Instrumentensatz nach einem der vorangehenden Ansprüche, **dadurch gekennzeichnet, daß** die Tulpe (20) an einem der ersten Einführöffnung (21a) entgegengesetzten zweiten Endbereich (25b) eine zweite Einführöffnung (21b) mit einem Randbereich (26) aufweist in dem zwei sich diametral gegenüberliegende Einbuchtungen (27) zur Aufnahme eines Stabes (101) des Stabsystems (100) ausgebildet sind.

15. Instrumentensatz nach Anspruch 14, **dadurch gekennzeichnet, daß** der Sattel (30) an einem in Richtung der zweiten Einführöffnung (21b) der Tulpe (20) angeordneten zweiten Endbereich (31b) eine sich diametral durch den Sattel (30) erstreckende Aussparung (39) zur Aufnahme des in den Einbuchtungen (27) des Randbereichs (26) der Tulpe angeordneten Stabes (101) aufweist.

16. Instrumentensatz nach einem der vorangehenden Ansprüche, **dadurch gekennzeichnet, daß** die Tulpe (20) an dem zweiten Endbereich (25b) eine Arretierungsvorrichtung (28) zur Fixierung des Stabes (101) aufweist.

17. Instrumentensatz nach einem der vorangehenden Ansprüche, **dadurch gekennzeichnet, daß** das Kopplungsteil (42) zylinderförmig ausgebildet ist.

18. Instrumentensatz nach einem der vorangehenden Ansprüche, **dadurch gekennzeichnet, daß** das Kopplungsteil (42) an einem dem ersten Endbereich (45a) entgegengesetzten zweiten Endbereich (45b) einen Drehgriff (46) aufweist.

19. Instrumentensatz nach einem der vorangehenden Ansprüche, **dadurch gekennzeichnet, daß** das Kopplungsteil (42) gegenüber dem Haltegriff (43) in axialer Richtung zwischen einer ersten Endlage (47a) und einer zweiten Endlage (47b) verschiebbar ist.

20. Instrumentensatz nach einem der vorangehenden Ansprüche, **dadurch gekennzeichnet, daß** die Schraube (10) massiv oder kanüliert und/oder gefenstert ist.

21. Instrumentensatz nach einem der vorangehenden Ansprüche, **dadurch gekennzeichnet, daß** die Schraube ein erstes Gewinde (17a) mit einer ersten Gewindesteigung und ein zweites Gewinde (17b) mit einer zweiten Gewindesteigung aufweist, und die erste und die zweite Gewindesteigung gleich oder verschieden sind.

22. Instrumentensatz nach Anspruch 21, **dadurch gekennzeichnet, daß** die erste Gewindesteigung und die zweite Gewindesteigung gleich sind und die beiden Gewinde (17a, b) derart entlang des Schaftes (14) der Schraube (10) angeordnet sind, daß die Schraube (10) in einem an den Schraubenkopf (12) angrenzenden zweiten Schaftbereich (16b) zweigängig ist und in einem sich von dem zweiten Schaftbereich (16b) entgegengesetzt zum Schraubenkopf (12) erstreckenden ersten Schaftbereich (16a) eingängig ist.

23. Instrumentensatz nach einem der vorangehenden Ansprüche, **dadurch gekennzeichnet, daß** der Schraubenkopf (12) eine Aussparung (18) zur Aufnahme eines Schraubendrehers (60) aufweist.

## Claims

1. An instrument set (1) for connecting vertebral bodies, comprising: a screw (10) which has a screw head (12) and a shaft (14) and is screwable into a pedicle; a top piece (2) which is connectable polyaxially to the screw (10) for coupling to a rod system (100) and which has a sleeve-shaped tulip (20) having a first insertion opening (21a) for inserting the screw head (12) in an insertion direction (E); and a tool (40); wherein the top piece (2) has a saddle (30) in the interior region (22) of the tulip (20), the saddle (30) is to be placed in the tulip (20) so that it is rotationally fixed and has a receiving region (32) for receiving the screw head (12) in a first end region (31a) that is facing the first insertion opening (21a) and a coupling region (35) to be coupled to the tool (40) in a second end region (31b) opposite the receiving region (32), and the saddle is movable between a released position (23a), which is reachable by inserting the screw head (12), and a locked position (23b), which is reachable by moving the screw head (12) out of the released position (23a) in a direction opposite the insertion direction (E), wherein when the saddle is in the locked position (23b) the screw head (12) held in the receiving region (32) cannot be removed from the receiving region (32) and, using the tool (40) coupled to the coupling region (35), the saddle (30) can be fixed in a released position (23a) that enables the release of the screw head (12) from the receiving region (32) so that the screw (10) and the top piece (2) are separable,
**characterised in that** the coupling region (35) of the saddle (30) has an internal thread (38), the tool has a holding grip (43) with a hollow interior space (44) and a coupling member (42) for coupling to the coupling region (35) of the saddle (30), wherein the coupling member (42) is placed at least partially in the interior space (44) and is rotatable around a longitudinal axis (A), the coupling member (42) of the tool (40) has an external thread (41) in a first end region (45a) for screwing to the internal thread (38) of the coupling region (35) and the tool (40) has a locking device (49) with which the holding grip (43) is fixable to the tulip (20) in a rotationally fixed manner.

2. The instrument set in accordance with claim 1, **characterised in that** the receiving region (32), which is delimited by a wall (32a) and has a receiving opening (32b) for inserting the screw head (12), is reversibly deformable in a transverse direction (Q) that is perpendicular to the longitudinal axis (L) of the tulip (20).

3. The instrument set in accordance with claim 2, **characterised in that** the receiving region (32) is elastically deformable.

4. The instrument set in accordance with claim 2 or 3, **characterised in that** the wall (32a) of the receiving region (32) is subdivided by at least two slots and/or windows (33) into at least two separate regions.

5. The instrument set in accordance with claim 4, **characterised in that** the slots and/or windows (33) are T-shaped.

6. The instrument set in accordance with claims 2 to 5, **characterised in that**, at least in the locked position (23b), a wall (24) of the tulip (20) limits a deformation of the receiving region (32) in the transverse direction (Q).

7. The instrument set in accordance with one of the preceding claims, **characterised in that**, in a first head region (15a), the screw head (12) has a maximum width (BK) in a direction perpendicular to the shaft (14), and a second head region (15b) located between the first head region (15a) and the shaft (14) has a second width (bK), in the direction perpendicular to the shaft (14), that is smaller than the maximum width (BK).

8. The instrument set in accordance with claims 6 and 7, **characterised in that**, in the locked position (23b) of the saddle (30), the wall (24) of the tulip (20) limits a maximum expansion of the receiving opening (32b) that can be achieved by a deformation in the transverse direction (Q) to a locked expansion (DS) that is smaller than the maximum width (BK) and greater than or equal to the second width (bK) of the screw head (12), and, in the released position (23a) of the saddle (30), the wall of the tulip (20) permits a maximum expansion of the receiving opening (32b) that can be achieved by a deformation in the transverse direction (Q) to a released expansion (DF) that is greater than the maximum width (BK) of the screw head (12).

9. The instrument set in accordance with one of the preceding claims, **characterised in that** an inner diameter (DImin) of the tulip (20) is at its minimum at the first insertion opening (21), and an inner diameter (DImax) of the tulip (20) is at its maximum in a plane (23c) defined by the receiving opening (32b) of the saddle (30) in the released position (23a).

10. The instrument set in accordance with one of the preceding claims, **characterised in that** the axial movement of the saddle (30) in the tulip (20) is limited between a first position (L1) and a second position (L2).

11. The instrument set in accordance with claim 10, **characterised in that** the first position (L1) coincides with the released position (23a).

12. The instrument set in accordance with claim 10 or 11, **characterised in that** the saddle (30) has at least one projection (37) between the receiving region (32) and the coupling region (35), projecting towards the wall (24) and engaging in a recess (24a) that is formed in the wall (24) of the tulip (20), wherein said projection (37), which abuts against a first and a second stop region (24b, c) that delimit the recess (24a) in the axial direction when the saddle (30) is moved axially, limits the movement of the saddle (30) between the first position (L1) and the second position (L2).

13. The instrument set in accordance with one of the preceding claims, **characterised in that** the tulip (20) and the saddle (30) are removably connected to each other.

14. The instrument set in accordance with one of the preceding claims, **characterised in that** the tulip (20) has, in a second end region (25b) opposite the first insertion opening (21a), a second insertion opening (21b) with an edge region (26) in which two diametrically opposite indentations (27) for receiving a rod (101) of the rod system (100) are formed.

15. The instrument set in accordance with claim 14, **characterised in that** the saddle (30) has, in a second end region (31b) located in the direction of the second insertion opening (21b) of the tulip (20), a recess (39) extending diametrically through the saddle (30) for receiving the rod (101) placed in the indentations (27) of the edge region (26) of the tulip.

16. The instrument set in accordance with one of the preceding claims, **characterised in that** the tulip (20) has a locking device (28) in the second end region (25b) for fixing the rod (101).

17. The instrument set in accordance with one of the preceding claims, **characterised in that** the coupling member (42) is cylindrical in shape.

18. The instrument set in accordance with one of the preceding claims, **characterised in that** the coupling member (42) has a twist grip (46) in a second end region (45b) opposite the first end region (45a).

19. The instrument set in accordance with one of the preceding claims, **characterised in that** the coupling member (42) is movable relative to the holding grip (43) in the axial direction between a first end position (47a) and a second end position (47b).

20. The instrument set in accordance with one of the preceding claims, **characterised in that** the screw (10) is solid or canulated and/or fenestrated.

21. The instrument set in accordance with one of the preceding claims, **characterised in that** the screw has a first thread (17a) having a first pitch and a second thread (17b) having a second pitch, and the first and second pitches are the same or different.

22. The instrument set in accordance with claim 21, **characterised in that** the first pitch and the second pitch arc the same and the two threads (17a, b) are arranged along the shaft (14) of the screw (10) so that the screw (10) is double-threaded in a second shaft region (16b) adjacent to the screw head (12) and is single-threaded in a first shaft region (16a) extending away from the second shaft region (16b) opposite the screw head (12).

23. The instrument set in accordance with one of the preceding claims, **characterised in that** the screw head (12) has a recess (18) for receiving a screwdriver (60).

## Revendications

1. Ensemble d'instruments (1) permettant d'unir des corps vertébraux, comprenant une vis (10) vissable dans un pédicule et présentant une tête (12) et une tige (14) ; un élément rapporté (2) susceptible d'être raccordé à la vis (10) de façon polyaxiale et destiné à être couplé à un système de tiges (100) et présentant une tulipe (20) tubulaire dotée d'une première ouverture d'introduction (21a) destinée à l'introduction de la tête de vis (12) dans une direction d'introduction (E) ; et un outil (40) ; ledit élément rapporté (2) présentant, dans la zone intérieure (22) de la tulipe (20), un socle (30), lequel socle (30) est susceptible d'être agencé immobile en rotation dans la tulipe (20) et présente, au niveau d'une première zone terminale (31a) tournée vers la première ouverture d'introduction (21a), une zone de réception (32) destinée à recevoir la tête de vis (12) et, au niveau d'une deuxième zone terminale (31b) opposée à la zone de réception (32), une zone de couplage (35) susceptible d'être couplée à l'outil (40), et lequel socle est mobile entre une position de dégagement (23a) susceptible d'être obtenue par introduction de la tête de vis (12) et une position de verrouillage (23b) d'être obtenue par décalage de la tête de vis (12) hors de la position de dégagement (23a) à l'opposé de la direction d'introduction (E), ladite tête de vis (12) reçue dans la zone de réception (32) en position de verrouillage (23b) n'étant pas retirable de la zone de réception (32), et le socle (30) pouvant être fixé grâce à l'outil (40), couplé à la zone de couplage (35), dans une position de dégagement (23a) permettant le dégagement de la tête de vis (12) hors de la zone de réception (32), de telle façon que la vis (10) et l'élément rapporté (2) sont séparables l'un de l'autre,
**caractérisé en ce que** la zone de couplage (35) du socle (30) présente un taraudage (38) ; l'outil présente un manche (43) doté d'un espace intérieur creux (44) ainsi qu'une pièce de couplage (42) destinée à un couplage à la zone de couplage (35) du socle (30), ladite pièce de couplage (42) étant agencée au moins partiellement dans l'espace intérieur (44) et étant rotative sur un axe longitudinal (A) ; la pièce de couplage (42) de l'outil (40) présente un filetage extérieur (41) dans une première zone terminale (45a) pour un vissage sur le taraudage (38) de la zone de couplage (35), et l'outil (40) présente un dispositif d'accroché (49) grâce auquel le manche (43) peut être raccordé immobile en rotation à la tulipe (20).

2. Ensemble d'instruments selon la revendication 1, **caractérisé en ce que** la zone de réception (32), délimitée par une paroi (32a) et présentant une ouverture de réception (32b) destinée à l'introduction de la tête de vis (12), est déformable de manière réversible dans une direction transversale (Q) perpendiculaire à un axe longitudinal (L) de la tulipe (20).

3. Ensemble d'instruments selon la revendication 2, **caractérisé en ce que** la zone de réception (32) est déformable élastiquement.

4. Ensemble d'instruments selon la revendication 2 ou 3, **caractérisé en ce que** la paroi (32a) de la zone de réception (32) est subdivisée en au moins deux zones distinctes l'une de l'autre par au moins deux fentes et/ou fenêtres (33).

5. Ensemble d'instruments selon la revendication 4, **caractérisé en ce que** les fentes et/ou fenêtres (33) sont en forme de T.

6. Ensemble d'instruments selon les revendications 2 à 5, **caractérisé en ce qu'**une paroi (24) de la tulipe (20) limite une déformation de la zone de réception (32) dans la direction transversale (Q), au moins dans la position de verrouillage (23b).

7. Ensemble d'instruments selon l'une des revendications précédentes, **caractérisé en ce que** la tête de vis (12) présente une largeur maximale (BK) dans une première zone de tête (15a) dans une direction perpendiculaire à la tige (14) et une deuxième zone de tête (15b) agencée entre la première zone de tête (15a) et la tige (14) présente une deuxième largeur (bK), dans une direction perpendiculaire à la tige (14), qui est inférieure à la largeur maximale (BK).

8. Ensemble d'instruments selon les revendications 6 et 7, **caractérisé en ce que**, dans la position de verrouillage (23b) du socle (30), la paroi (24) de la tulipe (20) limite une étendue maximale de l'ouverture de réception (32b), susceptible de résulter d'une déformation dans la direction transversale (Q), à une étendue de verrouillage (DS) qui est inférieure à la largeur maximale (BK) et supérieure ou égale à la deuxième largeur (bK) de la tête de vis (12) et, dans la position de dégagement (23a) du socle (30), la paroi de la tulipe (20) permet une étendue maximale de l'ouverture de réception (32b), susceptible de résulter d'une déformation dans la direction transversale (Q), à une étendue de dégagement (DF) qui est supérieure à la largeur maximale (BK) de la tête de vis (12).

9. Ensemble d'instruments selon l'une des revendications précédentes, **caractérisé en ce qu'**un diamètre intérieur (DImin) de la tulipe (20) est à son minimum au niveau de la première ouverture d'introduction (21) et un diamètre intérieur (DImax) de la tulipe (20) est à son maximum dans un plan (23c) défini par l'ouverture de réception (32b) du socle (30) dans la position de dégagement (23a).

10. Ensemble d'instruments selon l'une des revendications précédentes, **caractérisé en ce que** le mouvement axial du socle (30) dans la tulipe (20) est limité entre une première position (L1) et une deuxième position (L2).

11. Ensemble d'instruments selon la revendication 10, **caractérisé en ce que** la première position (L1) coïncide avec la position de dégagement (23a).

12. Ensemble d'instruments selon la revendication 10 ou 11, **caractérisé en ce que** le socle (30) présente, entre la zone de réception (32) et la zone de couplage (35), au moins une proéminence (37) faisant saillie vers la paroi (24) et pénétrant dans un évidement (24a) réalisé dans la paroi (24) de la tulipe (20), ladite proéminence (37) limitant le mouvement du socle (30) entre la première (L1) et la deuxième position (L2) lors d'un mouvement axial dudit socle (30) en venant buter contre une première et une deuxième zone de butée (24b, 24c) délimitant l'évidement (24a) dans le sens axial.

13. Ensemble d'instruments selon l'une des revendications précédentes, **caractérisé en ce que** la tulipe (20) et le socle (30) sont unis l'un à l'autre de manière amovible.

14. Ensemble d'instruments selon l'une des revendications précédentes, **caractérisé en ce que** la tulipe (20) présente, dans une deuxième zone terminale (25b) opposée à la première ouverture d'introduction (21a), une deuxième ouverture d'introduction (21b) dotée d'une zone de bordure (26) dans laquelle sont conçues deux échancrures (27) diamétralement opposées, destinées à recevoir une tige (101) du système de tiges (100).

15. Ensemble d'instruments selon la revendication 14, **caractérisé en ce que** le socle (30) présente, dans une deuxième zone terminale (31b) agencée vers la deuxième ouverture d'introduction (21b) de la tulipe (20), un évidement (39) s'étendant diamétralement dans le socle (30), destiné à recevoir la tige (101) agencée dans les échancrures (27) de la zone de bordure (26) de la tulipe.

16. Ensemble d'instruments selon l'une des revendications précédentes, **caractérisé en ce que** la tulipe (20) présente, dans la deuxième zone terminale (25b), un dispositif d'accroché (28) destiné à la fixation de la tige (101).

17. Ensemble d'instruments selon l'une des revendications précédentes, **caractérisé en ce que** la pièce de couplage (42) est de forme cylindrique.

18. Ensemble d'instruments selon l'une des revendications précédentes, **caractérisé en ce que** la pièce de couplage (42) présente une partie de préhension rotative (46), dans une deuxième zone terminale (45b) opposée à la première zone terminale (45a).

19. Ensemble d'instruments selon l'une des revendications précédentes, **caractérisé en ce que** la pièce de couplage (42) peut subir un mouvement de translation par rapport au manche (43) dans la direction axiale, entre une première position de butée (47a) et une deuxième position de butée (47b).

20. Ensemble d'instruments selon l'une des revendications précédentes, **caractérisé en ce que** la vis (10) est pleine ou canulée et/ou à fenêtre.

21. Ensemble d'instruments selon l'une des revendications précédentes, **caractérisé en ce que** la vis présente un premier filet (17a) avec un premier pas et un deuxième filet (17b) avec un deuxième pas, lesdits premier et deuxième pas étant identiques ou différents.

22. Ensemble d'instruments selon la revendication 21, **caractérisé en ce que** le premier pas et le deuxième pas sont identiques et les deux filets (17a, 17b) sont agencés le long de la tige (14) de la vis (10) de telle manière que la vis (10) présente un double pas dans une deuxième zone de tige (16b) contiguë à la tête de vis (12) et un pas unique dans une première zone de tige (16a) partant de la deuxième zone de tige (16b) en s'éloignant de la tête de vis (12).

23. Ensemble d'instruments selon l'une des revendications précédentes, **caractérisé en ce que** la tête de vis (12) présente un évidement (18) destiné à recevoir un tournevis (60).
